# EUROPEAN PATENT APPLICATION

(11) **EP 3 130 564 A1**
(43) Date of publication of application: **15.02.2017**
(21) Application number: 16190337.2
(22) Date of filing: 28.02.2013
(51) Int. Cl.: C02F 1/461, C02F 1/467, C25B 9/08, A61K 33/14, A61K 33/00

(54) **STABLE ELECTROLYZED SALINE SOLUTION**

(30) Priority: 21.09.2012 US 201261704401 P; 27.09.2012 US 201261706670 P; 28.09.2012 US 201261707141 P
(62) Divisional of application: 13839705.4
(71) Applicant: Reoxcyn Discoveries Group Inc., Salt Lake City, UT 84121 (US)
(72) Inventor: ROBINSON, Daniel, SALT LAKE CITY, UT Utah UT 84116 (US); PACK, James, Park City, UT Utah UT 84098 (US); SAMUELSON, Gary, SALT LAKE CITY, UT Utah UT 84121 (US)
(74) Representative: Dummett Copp LLP

(57) **Abstract**

A method for producing a stable, electrolyzed saline solution comprises:
preparing a saline solution having a ratio of about 10.75g of sodium chloride per 1 gallon (3.78541 litres) of water;
inserting within the saline solution an inert anode and a spaced apart corresponding inert cathode associated with a power source;
controlling the temperature of the saline solution to regulate relative concentrations of resulting species during electrolysis;
circulating the saline solution to maintain a flow of the saline solution between the anode and cathode;
applying a voltage potential selected from within a voltage range of 0 volts to approximately 12 volts between the cathode and the anode sufficient to produce a mixture of chemically reduced and oxidized molecules, the mixture including hypochlorous acid, hypochlorites, hydrogen gases, superoxides, ozone, chloride ions, hydroxides, and other forms of reactive oxygen species; and
mirroring molecular compositions of native salt water compounds found in and around human cells, said molecular compositions containing stable reactive oxygen species, said mirroring comprising one or more of the following:
adjusting a temperature of the saline solution;
adjusting anode and cathode spacing;
adjusting a saline solution circulation rate; and
optimization of the voltage.

## Description

### CROSS-REFERENCE TO RELATED APPLICATIONS

This application claims the benefit of U.S. Provisional Application No.'s 61/704,401, filed September 21, 2012; 61/706,670, filed September 27, 2012; and 61/707,141, filed September 28, 2012. Each of these provisional applications is incorporated by reference in its entirety.

### TECHNICAL FIELD

Compositions useful for the treatment of diseases related to oxidative stress and reduced mitochondrial DNA are disclosed.

### BACKGROUND

Virtually all biochemistry takes place in salt water, from the transcription of genes to the production of structures, proteins and enzymes. Considering this, it is not surprising that all life, from the most primitive prokaryotic bacteria to the most advanced eukaryotic mammalian cells (even plants), has developed the ability to oxidize and reduce salt water, thereby producing two classes of reactive molecules, namely the "reduced species" (RS) and "reactive oxygen species" (ROS). Both of these chemical species are produced from salt water and are prevalent in mammalian cells.

Mammalian cells utilize RS/ROS in a variety of biological processes. RS/ROS are produced in large amounts in the cell during the metabolism of sugar, specifically by the oxidative phosphorylation process in the mitochondria.

### SUMMARY

Embodiments include a composition comprising a redox signaling agent (RXN) and a Peroxisome Proliferator-Activated Receptor (PPAR) agonist. In an embodiment the PPAR agonist comprises a Free Fatty Acid (FFA). In an embodiment the PPAR agonist comprises an eicosanoid, or a thazolidinedione, or a fibrate. In an embodiment the PPAR agonist comprises a dual agonist such as aleglitazar, muraglitazar, or tesaglitazar. Embodiments include the use of a composition comprising RXN and a PPAR agonist in the treatment of a PPAR-mediated disease such as diabetes mellitus, including type 1 diabetes, type 2 diabetes, gestational diabetes, obesity, or cancer. Embodiments useful in the treatment of cancer can comprise a first therapy which activates a PPAR pathway and at least one other agent wherein the at least one other agent does not activate a PPAR pathway, such as radiation or a chemotherapeutic agent. Embodiments useful in the treatment of cancer can comprise a first therapy which mobilizes Free Fatty Acids (FFAS) and at least one other agent wherein the at least one other agent does not mobilize FFAs, such as radiation.

Embodiments include a method of treating an oxidative stress related disorder, such method including administering a composition including at least one species selected from O₂, H₂, Cl₂, OCr, HOCl, NaOCl, HClO₂, ClO₂, HClO₃, HClO₄, H₂O₂, Na+, Cr, H⁺, H⁻, OH⁻, O₃, O₄*⁻, ¹O, OH*⁻, HOCl-O₂*⁻, HOCl-O₃, O₂*⁻, HO₂*, NaCl, HCl, NaOH, water clusters, or a combination thereof to a patient experiencing oxidative stress; and treating the oxidative stress related disorder.

Embodiments include a method of treating a reduced mitochondrial DNA disorder comprising administering a composition including at least one species selected from O₂, H₂, Cl₂, OCr, HOC1, NaOCl, HClO₂, ClO₂, HClO₃, HClO₄, H₂O₂, Na⁺, Cl⁻, H⁺, H⁻, OH⁻, O₃, O₄*⁻, ¹O, OH*⁻, HOCl-O₂*⁻, HOCl⁻O₃, O₂*⁻, HO₂*, NaCl, HCl, NaOH, water clusters, or a combination thereof to a patient experiencing the reduced mitochondrial DNA disorder; increasing mitochondrial DNA density; and treating the reduced mitochondrial DNA disorder. Such disorders include sacropenia, diabetes, Alzheimer's disease, Parkinson's disease, neurological disease, muscle loss due to aging, obesity, or cardiovascular disorders.

### Brief Description of the Drawings

Figure 1 is a flow chart of a process as described herein.

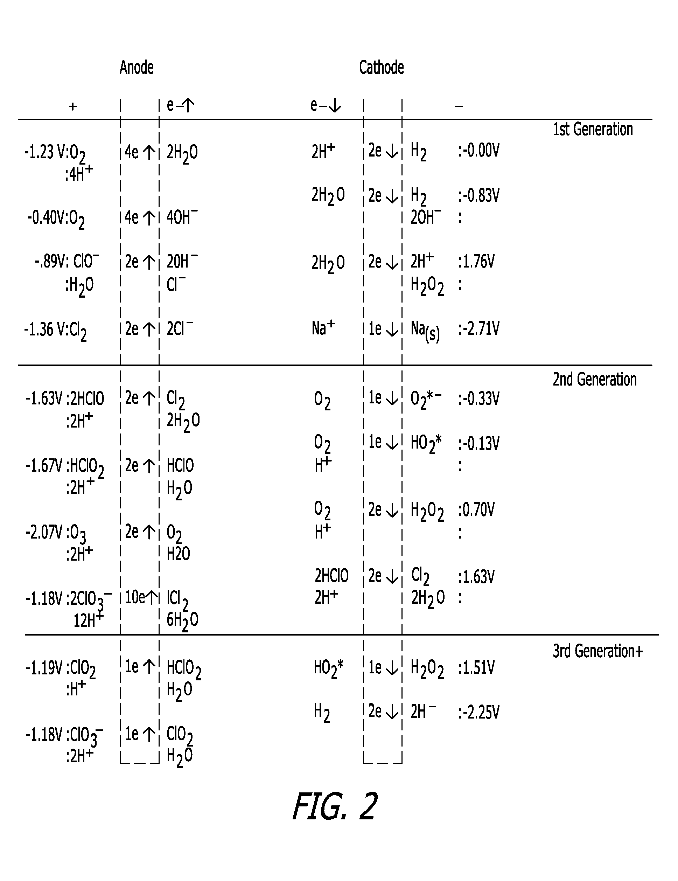

Figure 2 illustrates an example diagram of the generation of various molecules at the electrodes. The molecules written between the electrodes depict the initial reactants and those on the outside of the electrodes depict the molecules/ions produced at the electrodes and their electrode potentials.
Figure 3 illustrates a plan view of a process and system for producing a composition according to the present description.
Figure 4 illustrates an example system for preparing water for further processing into a composition described herein.
Figure 5 illustrates a C135 spectrum of a NaCl, NaClO solution at a pH of 12.48, and a composition described herein (the composition is labeled "ASEA").
Figure 6 illustrates a ¹H NMR spectrum of a composition of the present disclosure.
Figure 7 illustrates a 31P NMR spectrum of DIPPMPO combined with a composition described herein.
Figure 8 illustrates a mass spectrum showing a parent peak and fragmentation pattern for DIPPMPO with m/z peaks at 264, 222, and 180.
Figure 9 illustrates oxygen/nitrogen ratios for a composition described herein compared to water and NaClO (the composition is labeled "ASEA").
Figure 10 illustrates chlorine/nitrogen ratios for a composition described herein compared to water and NaClO (the composition is labeled "ASEA").
Figure 11 illustrates ozone/nitrogen ratios for a composition described herein compared to water and NaClO (the composition is labeled "ASEA").
Figure 12 illustrates the carbon dioxide to nitrogen ratio of a composition as described herein compared to water and NaClO (the composition is labeled "ASEA").
Figure 13 illustrates an EPR splitting pattern for a free electron.
Figure 14 illustrates a flow chart of the mouse study described in Example 3 (the composition-treatment group is labeled "ASEA").
Figure 15 is a flow chart showing a total overview of the mouse preparation and study (the composition used is referred-to as "ASEA").
Figure 16 illustrates mice grouped into placebo and ASEA (a composition described herein) treatment versus run time and versus glycogen depletion.
Figure 17A illustrates the fold change relate to ASEA (a composition described herein) treatment group of different mouse groups. Figure 17B illustrates the fold change difference between ASEA sedentary (non-running) and ASEA running groups.
Figure 18 illustrates different mouse groups versus the amount of liver Superoxide Dismutase (SOD) produced (the composition embodiment used is referred-to as "ASEA").
Figure 19A and 19B illustrate different mouse groups (sedentary/running; treatment/placebo) versus oxidized glutathione (the composition embodiment used is referred-to as "ASEA").
Figure 20 illustrates different mouse groups versus fold change for IL-6 and TNF-alpha (the composition embodiment used is referred-to as "ASEA").

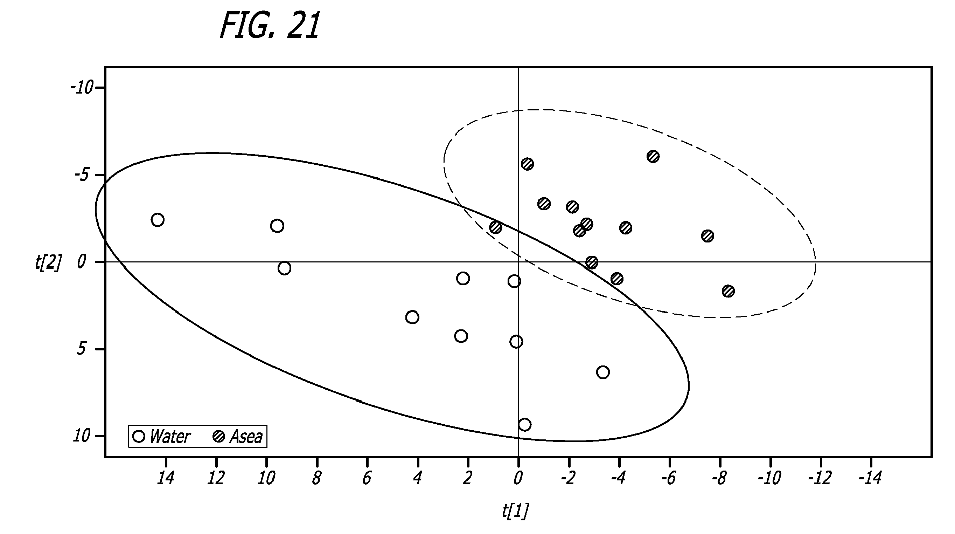

Figure 21 illustrates a comparison of A-B ratios between conditions 24 hours post ingestion (the composition embodiment used is referred-to as "ASEA").
Figure 22 illustrates a flow chart of the human running performance study protocol (the composition embodiment used in the protocol is referred-to as "ASEA").
Figure 23 illustrates a flow chart of a 12-week, randomized trial performed accord to the protocol of Example 7 (the composition embodiment used in the protocol is referred-to as "ASEA").
Figure 24 illustrates a graph of VCO₂ versus VO₂ resulting from the study in Example 8 (the composition embodiment used in the protocol is referred-to as "ASEA").
Figure 25 illustrates cell images for each culture results of HMVEC-L Cells p65 subunit NF-kB screen for toxicity (the composition embodiment used in the protocol is referred-to as "ASEA").
Figure 26 illustrates results for P-Jun screen for toxicity (the composition embodiment used in the protocol is referred-to as "ASEA").
Figure 27 illustrates a graph showing the reduction of oxidants over an 11 minute interval (RFU units on vertical scale).
Figure 28 illustrates a graph showing antioxidant activity over an 11 minute interval (the composition embodiment used in the protocol is referred-to as "ASEA").
Figure 29 illustrates nuclear staining patterns for results of HMVEC-L Nuclear Accumulation of NRF2 (the composition embodiment used in the protocol is referred-to as "ASEA").
Figure 30 illustrates serum-starved cell cultures exposed to low-concentration ASEA (a composition disclosed herein).
Figure 31 illustrates a western blot validation of NRF2 nuclear accumulation following ASEA treatment.

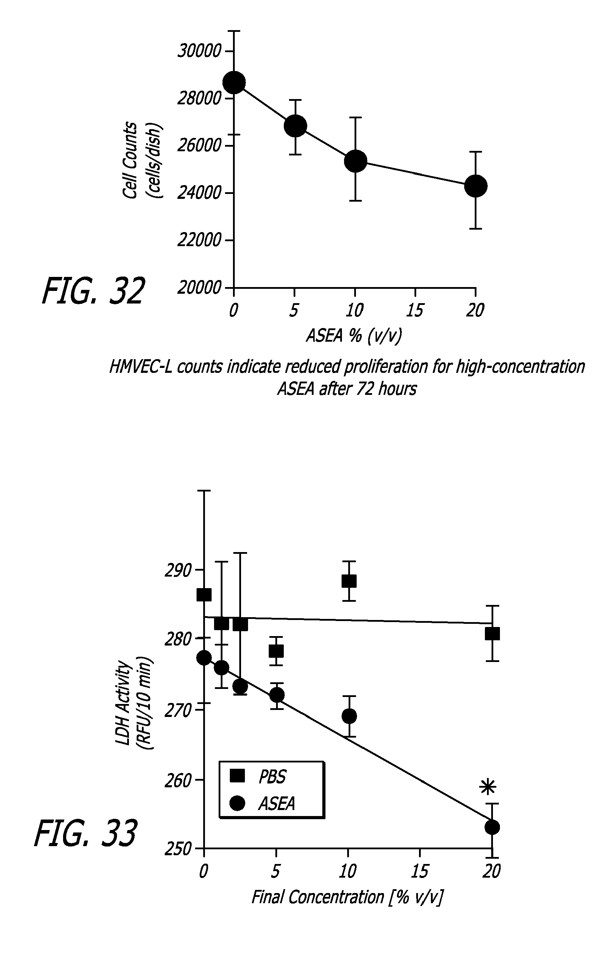

Figure 32 illustrates results for proliferation of murine and HMVEC-L cells and LDH activity following ASEA treatment.
Figure 33 illustrates further results for proliferation of murine and HMVEC-L cells and LDH activity following ASEA treatment.
Figure 34 illustrates results of HMVEC-L viability exposed high-concentration ASEA and to escalating amounts of Cachexin stressor (the composition embodiment used in the protocol is referred-to as "ASEA").
Figure 35 illustrates results of concentration-dependent response of HMVEC-L cells to Cachexin insult (the composition embodiment used in the protocol is referred-to as "ASEA").

### DETAILED DESCRIPTION

Described herein are therapeutic compositions that generally include at least one redox signaling agent (RXN). RXNs can include, but are not limited to superoxides: O₂*⁻, HO₂*; hypochlorites: OCr, HOC1, NaOCl; hypochlorates: HClO₂, ClO₂, HClO₃, HClO₄; oxygen derivatives: O₂, O₃, O₄*⁻, ¹O; hydrogen derivatives: H₂, H⁻; hydrogen peroxide: H₂O₂; hydroxyl free radicals: OH*⁻; ionic compounds: Na⁺, Cl⁻, H⁺, OH⁻, NaCl, HCl, NaOH; chlorine: Cl₂; and water clusters: n*H₂O - induced dipolar layers around ions. A composition comprising RXNs as described herein can mobilize free fatty acids (FFAs) and increase mitochondrial density. FFAs may activate Peroxisome Proliferator-Activated Receptors (PPARs).

Embodiments can further comprise a PPAR agonist. PPARs can be nuclear receptors. The PPAR family comprises three isoforms, designated alpha, gamma and delta (also called beta), each encoded by a different gene. These receptors, which form part of the superfamily of nuclear receptors and of transcription factors, may play a major role in regulation of lipid and carbohydrate metabolism. PPARs may play important roles in the regulation of cellular differentiation, development, and metabolism (carbohydrate, lipid, protein), and tumorigenesis of higher organisms.

PPAR-alpha may control lipid metabolism (hepatic and muscular) and glucose homeostasis, and may influence intracellular metabolism of lipids and sugars by direct control of transcription of the genes coding for proteins involved in lipid homeostasis. PPAR-alpha may also exert anti-inflammatory and antiproliferative effects and may prevent the proatherogenic effects of accumulation of cholesterol in macrophages by stimulating the outflow of cholesterol. PPAR-gamma is a key regulator of adipogenesis. It may also be involved in the lipid metabolism of mature adipocytes, in glucose homeostasis, in insulin resistance, in inflammation, in accumulation of cholesterol at the macrophage level and in cellular proliferation. PPAR-gamma consequently may play a role in the pathogenesis of obesity, insulin resistance and diabetes. PPAR-delta may be involved in controlling lipid and carbohydrate metabolism, in the energy balance, in neurodegeneration, in obesity, in the formation of foam cells and in inflammation.

Methods of making therapeutic compositions are described comprising: electrolyzing salinated water having a salt concentration of about 10 g NaCl/gal, such as 10.75 g NaCl/gal using a set of electrodes with an amperage of about 50-60 amps, such as 56 amps to form a life enhancing composition, wherein the water is chilled below room temperature and the water is circulated during electrolyzing.

A method of producing the disclosed compositions can include one or more of the steps of (1) preparation of an ultra-pure homogeneous solution of sodium chloride in water, (2) temperature control and flow regulation through a set of inert catalytic electrodes and (3) a modulated electrolytic process that results in the formation of such stable molecular moieties and complexes. In one embodiment, such a process includes all these steps.

The saline generally should be free from contaminants, both organic and inorganic, and homogeneous down to the molecular level. In particular, metal ions can interfere with the electro-catalytic surface reactions, and thus it may be helpful for metals to be avoided. In one embodiment, a brine solution is used to salinate the water. The brine solution can have a NaCl concentration of about 540 g NaCl/gal, such as 537.5 g NaCl/gal. In one embodiment, the composition can include at least one species such as O₂, H₂, Cl₂, OCr, HOC1, NaOCl, HClO₂, ClO₂, HClO₃, HClO₄, H₂O₂, Na⁺, Cl⁻, H⁺, H⁻, OH⁻, O₃, O₄*⁻, ¹O, OH*⁻, HOCl-O₂*⁻, HOCl-O₃ O²*⁻, HO₂*, NaCl, HCl, NaOH, water clusters, or a combination thereof.

In one embodiment, the composition can include at least one species such as H₂, Cl₂, OCr, HOC1, NaOCl, HClO₂, ClO₂, HClO₃, HClO₄, H₂O₂, O₃, O₄*⁻, ¹O₂, OH*⁻, HOCl-O₂*⁻, HOCl-O₃, O²*⁻, HO₂*, water clusters, or a combination thereof.

In one embodiment, the composition can include at least one species such as HClO₃, HClO₄, H₂O₂, O₃, O₄*⁻, ¹O₂, OH*⁻, HOCl-O²*⁻, HOCl-O₃, O₂*⁻, HO₂*, water clusters, or a combination thereof.

In one embodiment, the composition can include O₂. In one embodiment, the composition can include H₂. In one embodiment, the composition can include Cl₂. In one embodiment, the composition can include OCl⁻. In one embodiment, the composition can include HOCl. In one embodiment, the composition can include NaOCl. In one embodiment, the composition can include HClO₂. In one embodiment, the composition can include ClO₂. In one embodiment, the composition can include HClO₃. In one embodiment, the composition can include HClO₄. In one embodiment, the composition can include H₂O₂. In one embodiment, the composition can include Na⁺. In one embodiment, the composition can include Cl⁻. In one embodiment, the composition can include H⁺. In one embodiment, the composition can include H⁻. In one embodiment, the composition can include OH⁻. In one embodiment, the composition can include O₃. In one embodiment, the composition can include O₄*⁻. In one embodiment, the composition can include ¹O₂. In one embodiment, the composition can include OH*⁻. In one embodiment, the composition can include HOCl-O₂*⁻. In one embodiment, the composition can include HOCl-O₃, O₂*⁻. In one embodiment, the composition can include HO₂*. In one embodiment, the composition can include NaCl. In one embodiment, the composition can include HCl. In one embodiment, the composition can include NaOH. In one embodiment, the composition can include water clusters. Embodiments can include combinations thereof.

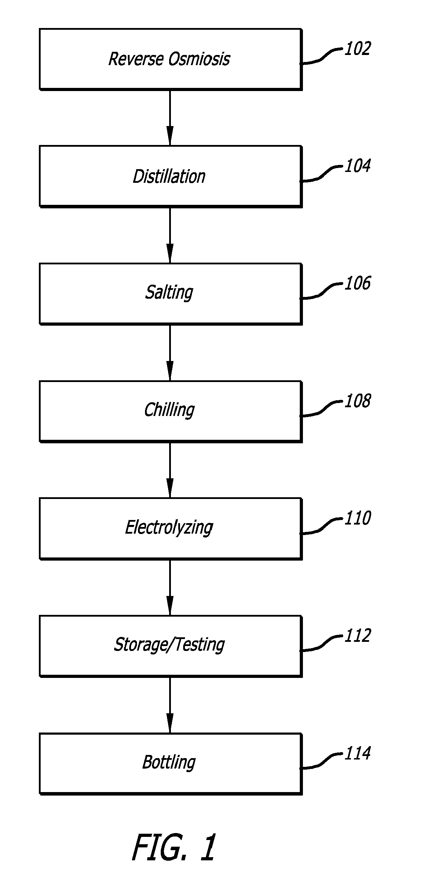

With this in mind, a step in such a process is shown in Figure 1. 100 is an optional reverse osmosis procedure 102. Water can be supplied from a variety of sources, including but not limited to municipal water, filtered water, nanopure water, or the like.

The reverse osmosis process can vary, but can provide water having a total dissolved solids content of less than about 10 ppm, about 9 ppm, about 8 ppm, about 7 ppm, about 6 ppm, about 5 ppm, about 4 ppm, about 3 ppm, about 2 ppm, about 1 ppm, or the like.

The reverse osmosis process can be performed at a temperature of about 5°C, about 10°C, about 15°C, about 20°C, about 25°C, about 30°C, about 35°C, or the like. The reverse osmosis step can be repeated as needed to achieve a particular total dissolved solids level. Whether the optional reverse osmosis step is utilized, an optional distillation step 104 can be performed.

The distillation process can vary, but can provide water having a total dissolved solids content of less than about 5 ppm, about 4 ppm, about 3 ppm, about 2 ppm, about 1 ppm, about 0.9 ppm, about 0.8 ppm, about 0.7 ppm, about 0.6 ppm, about 0.5 ppm, about 0.4 ppm, about 0.3 ppm, about 0.2 ppm, about 0.1 ppm, or the like. The temperature of the distillation process can be performed at a temperature of about 5°C, about 10°C, about 15°C, about 20°C, about 25°C, about 30°C, about 35°C, or the like.

The distillation step can be repeated as needed to achieve a particular total dissolved solids level. After water has been subjected to reverse osmosis, distillation, both, or neither, the level of total dissolved solids in the water can be less than about 5 ppm, about 4 ppm, about 3 ppm, about 2 ppm, about 1 ppm, about 0.9 ppm, about 0.8 ppm, about 0.7 ppm, about 0.6 ppm, about 0.5 ppm, about 0.4 ppm, about 0.3 ppm, about 0.2 ppm, about 0.1 ppm, or the like.

The reverse osmosis, distillation, both, or neither, can be preceded by a carbon filtration step.

Purified water can be used directly with the systems and methods described herein.

In one embodiment, contaminants can be removed from a commercial source of water by the following procedure: water flows through an activated carbon filter to remove the aromatic and volatile contaminants and then undergoes Reverse Osmosis (RO) filtration to remove dissolved solids and most organic and inorganic contaminants. The resulting filtered RO water can contain less than about 8 ppm of dissolved solids. Most of the remaining contaminants can be removed through a distillation process, resulting in dissolved solid measurements less than 1 ppm. In addition to removing contaminants, distillation may also serve to condition the water with the correct structure and Oxidation Reduction Potential (ORP) to facilitate the oxidative and reductive reaction potentials on the platinum electrodes in the subsequent electro-catalytic process.

After water has been subjected to reverse osmosis, distillation, both or neither, a salt is added to the water in a salting step 106. The salt can be unrefined, refined, caked, de-caked, or the like. In one embodiment, the salt is sodium chloride (NaCl). In some embodiments, the salt can include an additive. Salt additives can include, but are not limited to potassium iodide, sodium iodide, sodium iodate, dextrose, sodium fluoride, sodium ferrocyanide, tricalcium phosphate, calcium carbonate, magnesium carbonate, fatty acids, magnesium oxide, silicone dioxide, calcium silicate, sodium aluminosilicate, calcium aluminosilicate, ferrous fumarate, iron, or folic acid. Any of these additives can be added at this point or at any point during the described process. For example, the above additives can be added just prior to bottling.

Salt can be added to water in the form of a brine solution. To mix the brine solution, a physical mixing apparatus can be used or a circulation or recirculation can be used. In one embodiment, pure pharmaceutical grade sodium chloride is dissolved in the prepared distilled water to form a 15 wt % sub-saturated brine solution and continuously recirculated and filtered until the salt has completely dissolved and all particles > 0.1 microns are removed. This step can take several days. The filtered, dissolved brine solution is then injected into tanks of distilled water in about a 1:352 ratio (saltwater) in order to form a 0.3% saline solution. In one embodiment, a ratio 10.75 g of salt per 1 gallon of water can be used to form the composition. In another embodiment, 10.75 g of salt in about 3-4 g of water, such as 3.7875 g of water can be used to form the composition. This solution then can be allowed to re-circulate and diffuse until homogeneity at the molecular scale has been achieved.

In one embodiment, the homogenous saline solution is chilled to about 4.8±0.5°C. Temperature regulation during the entire electro-catalytic process can be employed as thermal energy generated from the electrolysis process itself may cause heating. In one embodiment, process temperatures at the electrodes can be constantly cooled and maintained at about 4.8°C throughout electrolysis.

Brine can then be added to the previously treated water or to fresh untreated water to achieve a NaCl concentration of between about 1 g NaCl/gal water and about 25 g NaCl/gal water, between about 8 g NaCl/gal water and about 12 g NaCl/gal water, or between about 4 g NaCl/gal water and about 16 g NaCl/gal water. Once brine is added to water at an appropriate amount, the solution can be thoroughly mixed. The temperature of the liquid during mixing can be at room temperature or controlled to a desired temperature or temperature range.

To mix the solution, a physical mixing apparatus can be used or a circulation or recirculation can be used. The salt solution can then be chilled in a chilling step 108.

For large amounts of composition, various chilling and cooling methods can be employed. For example cryogenic cooling using liquid nitrogen cooling lines can be used. Likewise, the solution can be run through propylene glycol heat exchangers to achieve the desired temperature. The chilling time can vary depending on the amount of liquid, the starting temperature and the desired chilled temperature.

Products from the anodic reactions can be effectively transported to the cathode to provide the reactants necessary to form the stable complexes on the cathode surfaces. Maintaining a high degree of homogeneity in the fluids circulated between the catalytic surfaces can also be helpful. A constant flow of about 2-8 mL/cm²*sec can be used, with typical mesh electrode distances 2 cm apart in large tanks. This flow can be maintained, in part, by the convective flow of gasses released from the electrodes during electrolysis.

The mixed solution, chilled or not, can then undergo electrochemical processing through the use of at least one electrode in an electrolyzing step 110. Each electrode can be or include a conductive metal. Metals can include, but are not limited to copper, aluminum, titanium, rhodium, platinum, silver, gold, iron, a combination thereof or an alloy such as steel or brass. The electrode can be coated or plated with a different metal such as, but not limited to aluminum, gold, platinum or silver. In an embodiment, each electrode is formed of titanium and plated with platinum. The platinum surfaces on the electrodes by themselves can be optimal to catalyze the required reactions. Rough, double layered platinum plating can assure that local "reaction centers" (sharply pointed extrusions) are active and that the reactants not make contact with the underlying electrode titanium substrate.

In one embodiment, rough platinum-plated mesh electrodes in a vertical, coaxial, cylindrical geometry can be optimal, with, for example, not more than 2.5 cm, not more than 5 cm, not more than 10 cm, not more than 20 cm, or not more than 50 cm separation between the anode and cathode. The amperage run through each electrode can be between about 2 amps and about 15 amps, between about 4 amps and about 14 amps, at least about 2 amps, at least about 4 amps, at least about 6 amps, or any range created using any of these values. In one embodiment, 7 amps is used with each electrode.

The amperage can be run through the electrodes for a sufficient time to electrolyze the saline solution. The solution can be chilled during the electrochemical process. The solution can also be mixed during the electrochemical process. This mixing can be performed to ensure substantially complete electrolysis.

Electric fields between the electrodes can cause movement of ions. Negative ions can move toward the anode and positive ions toward the cathode. This can enable exchange of reactants and products between the electrodes. In some embodiments, no barriers are needed between the electrodes.

In the mitochondria, fluctuations of the mitochondrial potential, specifically pulsing of the potentials have been seen to take place. Similarly, pulsing potentials in the power supply of the production units of compositions disclosed herein can also be utilized. Lack of filter capacitors in the rectified power supply can cause the voltages to drop to zero 120 times per second, resulting in a hard spike when the alternating current in the house power lines changes polarity. This hard spike, under Fourier transform, can emit a large bandwidth of frequencies. In essence, the voltage is varying from high potential to zero 120 times a second.

After amperage has been run through the solution for a sufficient time, an electrolyzed solution is created. The solution can be stored and or tested for particular properties in storage/testing step 112.

The end products of this electrolytic process can react within the saline solution to produce many different chemical entities. The compositions described herein can include one or more of these chemical entities, known as redox signaling agents or RXNs.

The chlorine concentration of the electrolyzed solution can be between about 5 ppm and about 34 ppm, between about 10 ppm and about 34 ppm, or between about 15 ppm and about 34 ppm. In one embodiment, the chlorine concentration is about 32 ppm.

The saline concentration in the electrolyzed solution can be, for example, between about 0.10% w/v and about 0.20% w/v, between about 0.11 % w/v and about 0.19% w/v, between about 0.12% w/v and about 0.18% w/v, between about 0.13% w/v and about 0.17% w/v, or between about 0.14% w/v and about 0.16% w/v.

The composition generally can include electrolytic and/or catalytic products of pure saline that mimic redox signaling molecular compositions of the native salt water compounds found in and around human cells. The composition can be fine tuned to mimic or mirror molecular compositions of different biological media. The composition can have reactive species other than chlorine present. As described, species present in the compositions described herein can include, but are not limited to O₂, H₂, Cl₂, OCr, HOC1, NaOCl, HClO₂, ClO₂, HClO₃, HClO₄, H₂O₂, Na⁺, Cr, H⁺, H⁻, OH⁻, O₃, O₄*⁻, ¹O₂, OH*⁻, HOCl-O₂*⁻, HOCl-O³, O₂*⁻, HO₂*, NaCl, HCl, NaOH, and water clusters: n*H₂O - induced dipolar layers around ions, and the like.

Compositions disclosed herein can also include PPAR agonists, such as, for example, FFAs, fibrates (fenofibrate, bezafibrate, ciprofibrate, gemfibrozil), thiazolidinediones (rosiglitazone and pioglitazone), L-165041, GW501516, KD3010, eicosanoids, prostaglandins (E1 - alprostadil, I2 - prostacyclin, PGJ2), prostacyclins (epoprostenol, treprostinil, FLOLAN®, veletri, remodulin, ventavis (iloprost), the thromboxanes (thromboxane A2, thromboxane B2), leukotrienes (L TC4, L TD4, L TE4 and L TF4 ),. perfluorooctanoic acid, perfluorononanoic acid, Berberine, RS5444, and the like. The PPAR agonist can be "dual", "balanced" or "pan" PPAR ligands ("glitazars"), including, for example, aleglitazar, muraglitazar, tesaglitazar, AM3102, CAY10506, CP 775146, DRF 2519, (+)-etomoxir sodium salt hydrate, GSK 3787, GW0742, GW 1929, GW 7647, GW1929 hydrate, W501516, L-165041, methyl-8-hydroxy-8-(2-pentyl-oxyphenyl)-oct-5-ynoate, NPC 15199, nTZDpa, PAz-PC, pioglitazone, rosiglitazone (potassium salt), rosiglitazone-d3 maleate, S26948, WY 14643, and the like.

In embodiments, the PPAR agonist can be packaged separately from the RXN composition comprising at least one RXN. For example, in an embodiment the PPAR agonist can be added to the RXN composition just prior to administration to a patient. In embodiments the PPAR agonist and the RXN can be administered in separate compositions. In embodiments the PPAR agonist can be contained within microspheres suspended within a RXN composition. In embodiments the PPAR agonist I RXN composition can comprise a gelcap.

Administration of PPAR agonist and/or RXN compositions can be achieved via any suitable method, including, for example, parenterally, by injection, epicutaneous, inhalational, enema, eye drops, ear drops, through mucous membranes in the body, by mouth (orally), gastric feeding tube, duodenal feeding tube, gastrostomy, rectally, intravenous, intra-arterial, intraosseous infusion, intra-muscular, intracerebral, intracerebroventricular, subcutaneous, or the like.

Compositions of the invention can be formulated into any suitable aspect, such as, for example, aerosols. liquids, elixirs, syrups, tinctures, creams, ointments, lotions, thin films, solids, gelcaps, a microsphere suspension, a soft gelatin capsule, and the like.

When administered as a liquid composition, it can be taken once, twice, three times, four times or more a day. Each administration can be about 1 oz, about 2 oz, about 3 oz, about 4 oz, about 5 oz, about 6 oz, about 7 oz, about 8 oz, about 9 oz, about 10 oz, about 11 oz, about 12 oz, about 16 oz, about 20 oz, about 24 oz, about 28 oz, about 32 oz, about 34 oz, about 36 oz, about 38 oz, about 40 oz, about 46 oz, between about 1 oz and about 32 oz, between about 1 oz and about 16 oz, between about 1 oz and about 8 oz, at least about 2 oz, at least about 4 oz, or at least about 8 oz. In one embodiment, the composition can be administered at a rate of about 4 oz twice a day.

In other embodiments, the administration can be acute or long term. For example, the composition can be administered for a day, a week, a month, a year or longer.

The compositions described herein when administered can be used to treat a condition or a disease. For example, when administered alongside exercise or not, the compositions described herein can increase the density of mitochondrial DNA. For example, an increase in mitochondrial DNA of about 1 %, about 5%, about 10%, about 15%, about 20%, about 21 %, about 22%, about 23%, about 24%, about 25%, about 26%, about 27%, about 28%, about 29%, about 30%, about 32%, about 34%, about 36%, about 38%, about 40%, about 45%, between about 1 % and about 40%, between about 1 % and about 10%, between about 20% and about 30%, at least about 5%, at least about 10%, or at least about 20% when compared to an individual who has not taken the composition. An increase in mitochondrial DNA can result in a lower level of free radicals in the blood which can in turn lead to a reduced amount of oxidative stress.

Compositions disclosed herein can be useful in treating diseases related to mitochondrial DNA. As such, the compositions described can treat conditions or diseases such as, but not limited to sacropenia, Parkinson's disease, neuro-related age disease, obesity, aging, life stresses such as those caused by fear, neurodegenerative diseases, cognitive disorders, obesity, reduced metabolic rate, metabolic syndrome, diabetes mellitus, cardiovascular disease, hyperlipidemia, neurodegenerative disease, cognitive disorder, mood disorder, stress, and anxiety disorder; for weight management, or to increase muscle performance or mental performance, AIDS, dementia complex, Alzheimer's disease, amyotrophic lateral sclerosis, adrenoleukodystrophy, Alexander disease, Alper's disease, ataxia telangiectasia, Batten disease, bovine spongiform encephalopathy (BSE), Canavan disease, corticobasal degeneration, Creutzfeldt-Jakob disease, dementia with Lewy bodies, fatal familial insomnia, frontotemporal lobar degeneration, Huntington's disease, Kennedy's disease, Krabbe disease, Lyme disease, Machado-Joseph disease, multiple sclerosis, multiple system atrophy, neuroacanthocytosis, Niemann-Pick disease, Pick's disease, primary lateral sclerosis, progressive supranuclear palsy, Refsum disease, Sandhoff disease, diffuse myelinoclastic sclerosis, spinocerebellar ataxia, subacute combined degeneration of spinal cord, tabes dorsalis, Tay-Sachs disease, toxic encephalopathy, transmissible spongiform encephalopathy, and wobbly hedgehog syndrome, cognitive function abnormalities, perception abnormalities, attention disorders, speech comprehension disorders, reading comprehension disorders, creation of imagery disorders, learning disorders, reasoning disorders, mood disorders, depression, postpartum depression, dysthymia, bipolar disorder, generalized anxiety disorder, panic disorder, panic disorder with agoraphobia, agoraphobia, social anxiety disorder, obsessive-compulsive disorder, posttraumatic stress disorder, musculoskeletal disorder, lack of strength, lack of endurance, cancer, atherosclerotic lesions, atherosclerosis, oxidative stress, atherogenesis, hypertension, hypercholesterolemia, and degenerative diseases.

Compositions disclosed herein can be useful in treating diseases involving PPAR pathways, including, for example, metabolic syndrome, cardiovascular disease, diabetes, obesity, glucose intolerance, hyperinsulinemia, hypercholesterolemia, hypertriglyceridemia, and hypertension, inflammation, vascular function, and vascular remodeling, cancer, inflammation, neurodegenerative diseases, diseases relating to mitochondrial biogenesis, ageing, and the like.

In embodiments, compositions of the invention can comprise a component of a therapy acting through multiple mechanisms. For example, a dual acting therapy can comprise a first therapy which activates a PPAR pathway and at least one other agent that does not activate a PPAR pathway. In embodiments the first agent can be at least one RXN. In embodiments the at least one other agent is a mAb, radiation, surgery, angiogenesis inhibitor, transplantation, a cancer vaccine, gene therapy, laser treatment, photodynamic therapy, an alkylating agent, an antimetabolite, an anti-tumor antibiotic, a topoisomerase inhibitor, a mitotic inhibitor, a corticosteroid, hormone therapy, immunotherapy, and the like.

Embodiments can provide a substrate in vitro for superoxides and/or fatty acid oxidation (FAQ) enzymes comprising RXNs. Embodiments can include a method of treating cancer comprising a combination therapy wherein one therapy comprises compositions comprising at least one RXN which increases FFAs, and at least one other agent wherein the at least one other agent does not increase FFAs.

In other embodiments, methods of treating an oxidative stress related disorders are described comprising: administering a composition including at least one species selected from O₂, H₂, Cl₂, OCr, HOC1, NaOCl, HClO₂, ClO₂, HClO₃, HClO₄, H₂O₂, Na⁺, Cr, H⁺, H⁻, OH⁻, O₃, O₄*⁻, ¹0, OH*⁻, HOCl-O₂*⁻, HOCl-O₃, O₂*⁻, HO₂*, NaCl, HCl, NaOH, water clusters, or a combination thereof to a patient experiencing oxidative stress; and treating the oxidative stress related disorder. In some embodiments, the administration occurs twice a day or once a day. Each administration can include between about 1 oz and about 16 oz per day. In other embodiments, the oxidative stress related disorder is diabetes, cardiovascular disease, or obesity.

### Example 1

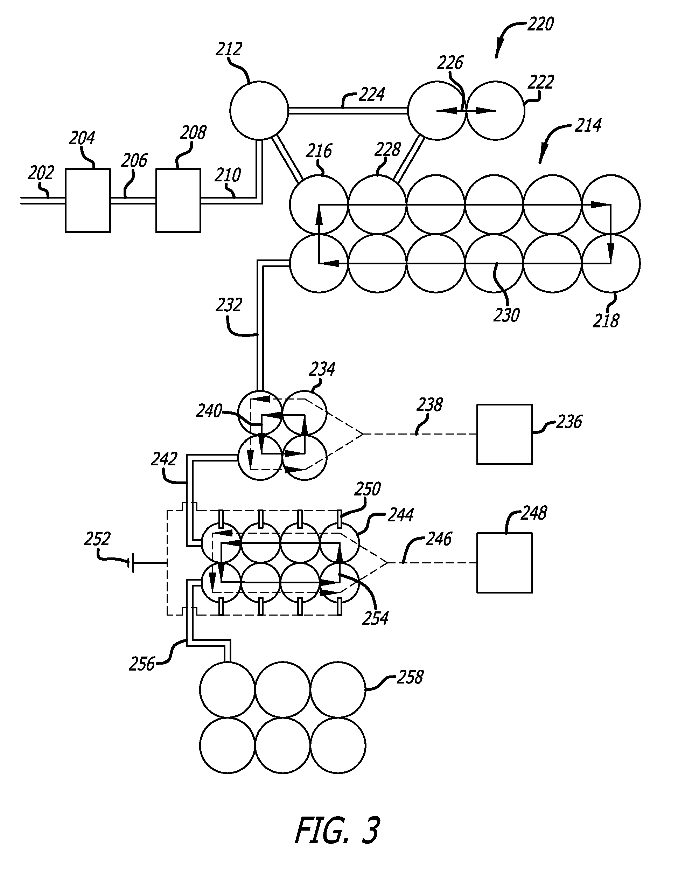

Figure 3 illustrates a plan view of a process and system for producing a life enhancing composition according to the present description. One skilled in the art understands that changes can be made to the system to alter the life enhancing composition, and these changes are within the scope of the present description.

Incoming water **202** can be subjected to reverse osmosis system **204** at a temperature of about 15-20°C to achieve purified water **206** with about 8 ppm of total dissolved solids. Purified water **206,** is then fed at a temperature of about 15-20°C into distiller **208** and processed to achieve distilled water **210** with about 0.5 ppm of total dissolved solids. Distilled water **210** can then be stored in tank **212.**

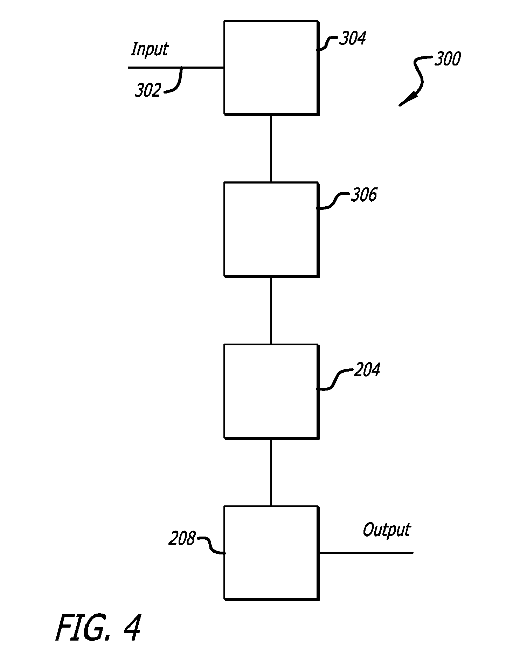

Figure 4 illustrates an example system for preparing water for further processing into a therapeutic beverage. System **300** can include a water source **3O2** which can feed directly into a carbon filter **304.** After oils, alcohols, and other volatile chemical residuals and particulates are removed by carbon filter **304,** the water can be directed to resin beds within a water softener **306** which can remove dissolved minerals. Then, as described above, the water can pass through reverse osmosis system **204** and distiller **208.**

As needed, distilled water **210** can be gravity fed from tank **212** into saline storage tank cluster **214** using line **216.** Saline storage tank cluster **214** in one embodiment can include twelve tanks **218.** Each tank **218** can be filled to about 1,300 gallons with distilled water **210.** A hand held meter can be used to test distilled water **210** for salinity.

Saline storage tank cluster **214** is then salted using a brine system **220.** Brine system **220** can include two brine tanks **222.** Each tank can have a capacity of about 500 gallons. Brine tanks **222** are filled to 4 75 gallons with distilled water **210** using line **224** and then NaCl is added to the brine tanks **222** at a ratio of about 537.5 g/gal of liquid. At this point, the water is circulated **226** in the brine tanks **222** at a rate of about 2,000 gal/hr for about 4 days.

Prior to addition of brine to tanks **218,** the salinity of the water in tanks **218** can be tested using a handheld conductivity meter such as an YSI ECOSENSE® ecp300 (YSI Inc., Yellow Springs, OH). Any corrections based on the salinity measurements can be made at this point. Brine solution **228** is then added to tanks **218** to achieve a salt concentration of about 10. 75 g/gal. The salted water is circulated **230** in tanks **218** at a rate of about 2,000 gal/hr for no less than about 72 hours. This circulation is performed at room temperature. A handheld probe can again be used to test salinity of the salinated solution. In one embodiment, the salinity is about 2.8 ppth.

In one method for filling and mixing the salt water in the brine holding tanks, the amount of liquid remaining in the tanks is measured. The amount of liquid remaining in a tank is measured by recording the height that the liquid level is from the floor that sustains the tank, in centimeters, and referencing the number of gallons this height represents. This can be done from the outside of the tank if the tank is semi-transparent. The initial liquid height in both tied tanks can also be measured. Then, after ensuring that the output valve is closed, distilled water can be pumped in. The amount of distilled water that is being pumped into a holding tank can then be calculated by measuring the rise in liquid level: subtracting the initial height from the filled height and then multiplying this difference by a known factor.

The amount of salt to be added to the tank is then calculated by multiplying 11 grams of salt for every Gallon of distilled water that has been added to the tank. The salt can be carefully weighed out and dumped into the tank.

The tank is then agitated by turning on the recirculation pump and then opening the top and bottom valves on the tank. Liquid is pumped from the bottom of the tank to the top. The tank can be agitated for three days before it may be ready to be processed.

After agitating the tank for more than 6 hours, the salinity is checked with a salinity meter by taking a sample from the tank and testing it. Salt or water can be added to adjust the salinity within the tanks. If either more water or more salt is added then the tanks are agitated for 6 more hours and tested again. After about three days of agitation, the tank is ready to be processed.

Salinated water **232** is then transferred to cold saline tanks **234.** In one embodiment, four 250 gal tanks are used. The amount of salinated water **232** moved is about 1,000 gal. A chiller **236** such as a 16 ton chiller is used to cool heat exchangers **238** to about 0-5°C. The salinated water is circulated **240** through the heat exchangers which are circulated with propylene glycol until the temperature of the salinated water is about 4.5-5.80C. Chilling the 1,000 gal of salinated water generally takes about 6-8 hr.

Cold salinated water **242** is then transferred to processing tanks **244.** In one embodiment, eight tanks are used and each can have a capacity of about 180 gal. Each processing tank **244** is filled to about 125 gal for a total of 1,000 gal. Heat exchangers **246** are again used to chill the cold salinated water **242** added to processing tanks **244.** Each processing tank can include a cylinder of chilling tubes and propylene glycol can be circulated. The heat exchangers can be powered by a 4-5 ton chiller **248.** The temperature of cold salinated water **242** can remain at 4.5-5.8°C during processing.

Prior to transferring aged salt water to processing tanks, the aged salt water can be agitated for about 30 minutes to sufficiently mix the aged salt water. Then, the recirculation valves can then be closed, the appropriate inlet valve on the production tank is opened, and the tank filled so that the salt water covers the cooling coils and comes up to the fill mark (approximately 125 gallons).

Once the aged salt water has reached production temperature, the pump is turned off but the chiller left on. The tank should be adequately agitated or re-circulated during the whole duration of electrochemical processing and the temperature should remain constant throughout.

Each processing tank **244** includes electrode **250.** Electrodes **250** can be 3 inches tall circular structures formed of titanium and plated with platinum. Electrochemical processing of the cold salinated water can be run for 8 hr. A power supply **252** is used to power the eight electrodes (one in each processing tank **244**) to 7 amps each for a total of 56 amps. The cold salinated water is circulated **254** during electrochemical processing at a rate of about 1,000 gal/hr.

An independent current meter can be used to set the current to around 7.0 Amps. Attention can be paid to ensure that the voltage does not exceed 12V and does not go lower than 9V. Normal operation can be about 10V.

A run timer can be set for a prescribed time (about 4.5 to 5 hours). Each production tank can have its own timer and/or power supply. Electrodes should be turned off after the timer has expired.

The production tanks can be checked periodically. The temperature and/or electrical current can be kept substantially constant. At the beginning, the electrodes can be visible from the top, emitting visible bubbles. After about 3 hours, small bubbles of undissolved oxygen can start building up in the tank as oxygen saturation occurs, obscuring the view of the electrodes. A slight chlorine smell can be normal.

After the 8 hour electrochemical processing is complete, life enhancing water **256** has been created with a pH of about 6.8-8.2, 32 ppm of chlorine, 100% OCl⁻ and 100% 0⁻². The composition **256** is transferred to storage tanks **258.**

### Example 2

### Characterization of a beverage produced as described in Example 1

A composition produced as described in Example 1 and marketed under the trade name ASEA® was analyzed using a variety of different characterization techniques. ICP/MS and 35Cl NMR were used to analyze and quantify chlorine content. Headspace mass spectrometry analysis was used to analyze adsorbed gas content in the beverage. 1 H NMR was used to verify the organic matter content in the beverage. 31 P NMR and EPR experiments utilizing spin trap molecules were used to explore the beverage for free radicals.

The composition was received and stored at about 4°C when not being used.

### Chlorine NMR

Sodium hypochlorite solutions were prepared at different pH values. 5% sodium hypochlorite solution had a pH of 12.48. Concentrated nitric acid was added to 5% sodium hypochlorite solution to create solutions that were at pH of 9.99, 6.99, 5.32, and 3.28. These solutions were then analyzed by NMR spectroscopy. The beverage had a measured pH of 8.01 and was analyzed directly by NMR with no dilutions.

NMR spectroscopy experiments were performed using a 400 MHz Bruker spectrometer equipped with a BBO probe. ³⁵Cl NMR experiments were performed at a frequency of 39.2 MHz using single pulse experiments. A recycle delay of 10 seconds was used, and 128 scans were acquired per sample. A solution of NaCl in water was used as an external chemical shift reference. All experiments were performed at room temperature.

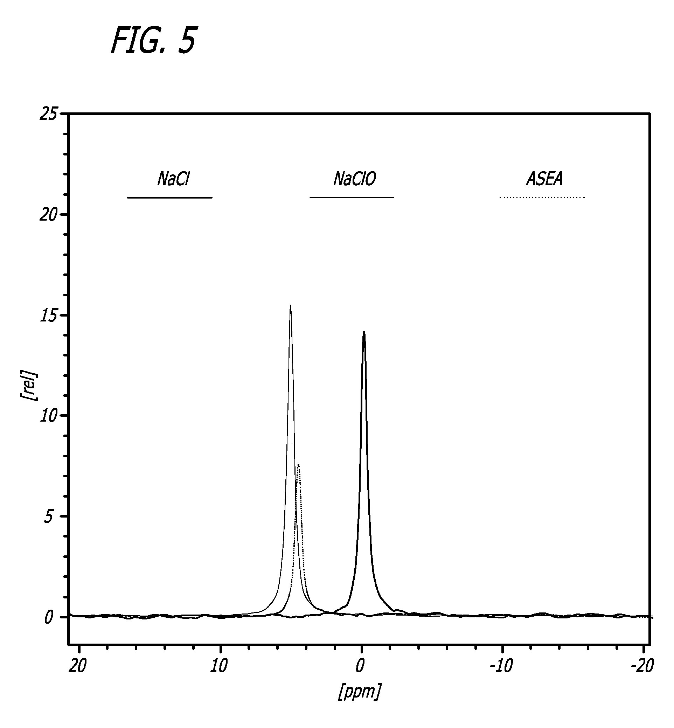

³⁵Cl NMR spectra were collected for NaCl solution, NaClO solutions adjusted to different pH values, and the composition. Figure 5 illustrates a C135 spectrum of NaCl, NaClO solution at a pH of 12.48, and the composition. The chemical shift scale was referenced by setting the Cl⁻ peak to 0 ppm. NaClO solutions above a pH=7 had identical spectra with a peak at approximately 5.1 ppm. Below pH of 7.0, the ClO⁻ peak disappeared and was replaced by much broader, less easily identifiable peaks. The composition was presented with one peak at approximately 4.7 ppm, from ClO⁻ in the composition. This peak was integrated to estimate the concentration of ClO⁻ in the composition, which was determined to be 2.99 ppt or 0.17 M of ClO⁻ in the composition.

### Proton NMR

An ASEA sample was prepared by adding 550 µL of ASEA and 50 µL of D₂O (Cambridge Isotope Laboratories) to an NMR tube and vortexing the sample for 10 seconds. 1H NMR experiments were performed on a 700 MHz Bruker spectrometer equipped with a QNP cryogenically cooled probe. Experiments used a single pulse with pre-saturation on the water resonance experiment. A total of 1024 scans were taken. All experiments were performed at room temperature.

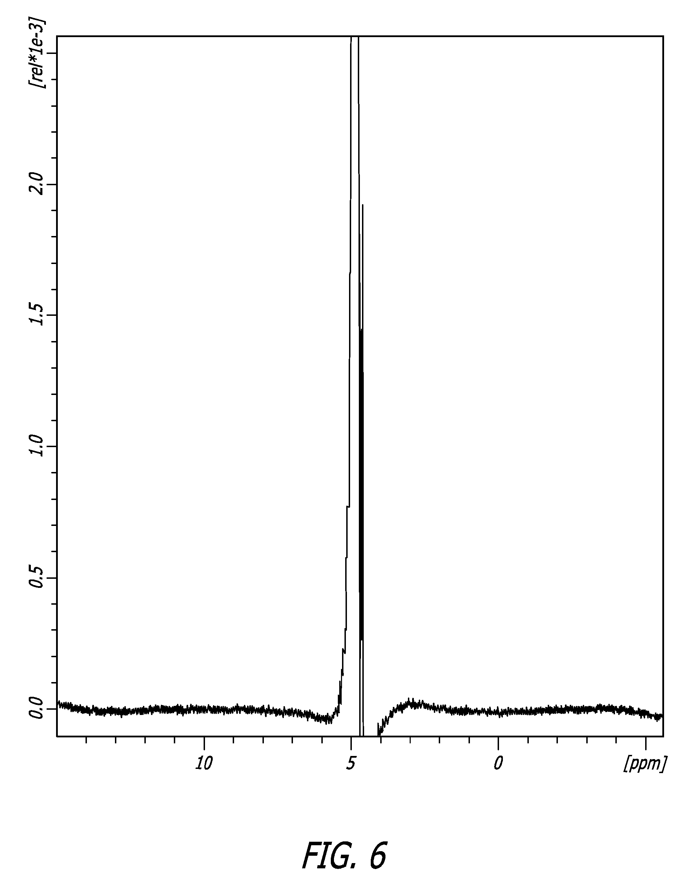

A ¹H NMR spectrum of the composition was determined and is presented in Figure 6. Only peaks associated with water were able to be distinguished from this spectrum. This spectrum show that very little if any organic material can be detected in the composition using this method.

### Phosphorous NMR and Mass Spectrometry

DIPPMPO (5-(Diisopropoxyphosphoryl)-5-1-pyrroline-N-oxide) (VWR) samples were prepared by measuring about 5 mg of DIPPMPO into a 2 mL centrifuge tube. This tube then had 550 µL of either the composition or water added to it, followed by 50 µL of D₂O. A solution was also prepared with the composition but without DIPPMPO. These solutions were vortexed and transferred to NMR tubes for analysis. Samples for mass spectrometry analysis were prepared by dissolving about 5 mg of DIPPMPO in 600 µL of the composition and vortexing, then diluting the sample by adding 100 µL of sample and 900 µL of water to a vial and vortexing.

NMR experiments were performed using a 700 MHz Bruker spectrometer equipped with a QNP cryogenically cooled probe. Experiments performed were a single 30° pulse at a ³¹P frequency of 283.4 MHz. A recycle delay of 2.5 seconds and 16384 scans were used. Phosphoric acid was used as an external standard. All experiments were performed at room temperature.

Mass spectrometry experiments were performed by directly injecting the ASEN/DIPPMPO sample into a Waters/Synapt Time of Flight mass spectrometer. The sample was directly injected into the mass spectrometer, bypassing the LC, and monitored in both positive and negative ion mode.

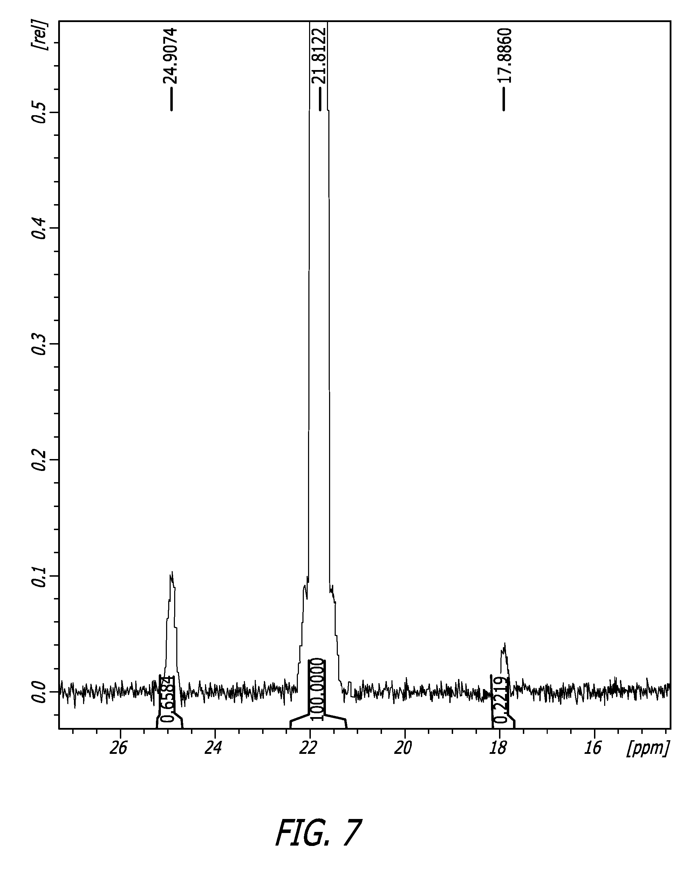

³¹P NMR spectra were collected for DIPPMPO in water, the composition alone, and the composition with DIPPMPO added to it. An external reference of phosphoric acid was used as a chemical shift reference. Figure 7 illustrates a ³¹P NMR spectrum of DIPPMPO combined with the composition. The peak at 21.8 ppm was determined to be DIPPMPO and is seen in both the spectrum of DIPPMPO with the composition (Figure 7) and without the composition (not pictured). The peak at 24.9 ppm is most probably DIPPMPO/OH• as determined in other DIPPMPO studies. This peak may be seen in DIPPMPO mixtures both with and without the composition, but is detected at a much greater concentration in the solution with the composition. In the DIPPMPO mixture with the composition, there is another peak at 17.9 ppm. This peak may be from another radical species in the composition such as OOH• or possibly a different radical complex. The approximate concentrations of spin trap complexes in the composition /DIPPMPO solution are as follows:

| **Solution** | **Concentration** |
|---|---|
| DIPPMPO | 36.6 mM |
| DIPPMPO/OH• | 241 µM |
| DIPPMPO/ radical | 94 µM |

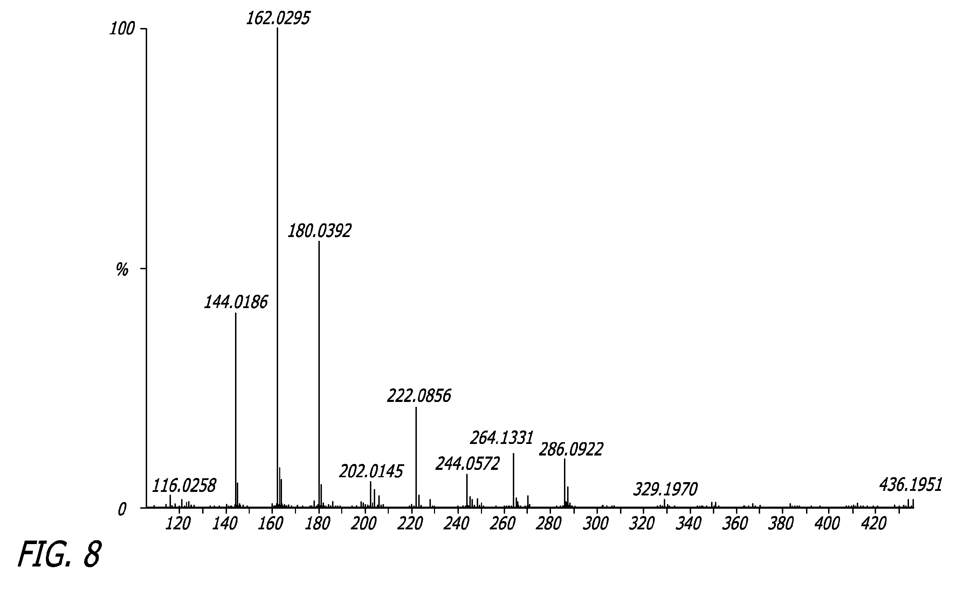

Mass spectral data was collected in an attempt to determine the composition of the unidentified radical species. The mass spectrum shows a parent peak and fragmentation pattern for DI PPM PO with m/z peaks at 264, 222, and 180, as seen in Figure 8. Figure 8 also shows peaks for the DIPPMPO/Na adduct and subsequent fragments at 286, 244, and 202 m/z. Finally, Figure 8 demonstrates peaks for one DIPPMPO/radical complex with m/z of 329. The negative ion mode mass spectrum also had a corresponding peak at m/z of 327. There are additional peaks at 349, 367, and 302 at a lower intensity as presented in Figure 8. None of these peaks could be positively confirmed. However, there are possible structures that would result in these mass patterns. One possibility for the peak generated at 329 could be a structure formed from a radical combining with DIPPMPO. Possibilities of this radical species include a nitroxyl-peroxide radical (HNO-HOO•) that may have formed in the beverage as a result of reaction with nitrogen from the air. Another peak at 349 could also be a result of a DIPPMPO/radical combination. Here, a possibility for the radical may be hypochlorite-peroxide (HOCl⁻HOO•). However, the small intensity of this peak and small intensity of the corresponding peak of 347 in the negative ion mode mass spectrum indicate this could be a very low concentration impurity and not a compound present in the ASEA composition.

### ICP/MS Analysis

Samples were analyzed on an Agilent 7500 series inductively-coupled plasma mass spectrometer (ICP-MS) in order to confirm the hypochlorite concentration that was determined by NMR. A stock solution of 5% sodium hypochlorite was used to prepare a series of dilutions consisting of 300 ppb, 150 ppb, 75 ppb, 37.5 ppb, 18.75 ppb, 9.375 ppb, 4.6875 ppb, 2.34375 ppb, and 1.171875 ppb in deionized Milli-Q water. These standards were used to establish a standard curve.

Based on NMR hypochlorite concentration data, a series of dilutions was prepared consisting of 164.9835 ppb, 82.49175 ppb, 41.245875 ppb, 20.622937 ppb, 10.311468 ppb, and 5.155734 ppb. These theoretical values were then compared with the values determined by ICP-MS analysis. The instrument parameters were as follows:

| | |
|---|---|
| **Elements analyzed** | ³⁵Cl, ³⁷Cl |
| **# of points per mass** | 20 |
| **# of repetitions** | 5 |
| **Total acquisition time** | 68.3s |
| **Uptake speed** | 0.50 rps |
| **Uptake time** | 33s |
| **Stabilization time** | 40s |
| **Tune** | No Gas |
| **Nebulizer flow rate** | 1 mL/min |
| **Torch power** | 1500 W |

The results of the ICP-MS analysis are as follows:

| **Dilution** | **Measured Concentration (ppb)** | **Concentration by NMR (ppb)** |
|---|---|---|
| 1 | 81 | 82 |
| 2 | 28 | 41 |
| 3 | 24 | 21 |
| 4 | 13 | 10 |
| 5 | 8 | 5 |

Dilutions were compared graphically to the ICP-MS signals and fit to a linear equation (R2=0.9522). Assuming linear behavior of the ICP-MS signal, the concentration of hypochlorite in the beverage was measured to be 3.02 ppt. Concentration values were determined by calculating the concentration of dilutions of the initial beverage and estimating the initial beverage hypochlorite concentration to be 3 ppt (as determined from 35Cl NMR analysis). The ICP-MS data correlate well with the 35Cl NMR data, confirming a hypochlorite concentration of roughly 1/3% (3 ppt). It should be noted that ICP-MS analysis is capable of measuring total chlorine atom concentration in solution, but not specific chlorine species. The NMR data indicate that chlorine predominantly exists as Clo- in the beverage.

### Gas Phase Quadrupole MS

### Sample Prep

Three sample groups were prepared in triplicate for the analysis: 1) Milli-Q deionized water 2) the composition, and 3) 5% sodium hypochlorite standard solution. The vials used were 20 mL headspace vials with magnetic crimp caps (GERSTEL). A small stir bar was placed in each vial (VWR) along with 10 mL of sample. The vials were capped, and then placed in a Branson model 5510 sonicator for one hour at 60°C.

The sonicator was set to degas which allowed for any dissolved gasses to be released from the sample into the headspace. After degassing, the samples were placed on a CTC PAL autosampler equipped with a heated agitator and headspace syringe. The agitator was set to 750 rpm and 95°C and the syringe was set to 75°C. Each vial was placed in the agitator for 20 min prior to injection into the instrument. A headspace volume of 2.5 mL was collected from the vial and injected into the instrument.

### Instrument Parameters

The instrument used was an Agilent 7890A GC system coupled to an Agilent 5975C El/Cl single quadrupole mass selective detector (MSD) set up for electron ionization. The GC oven was set to 40°C with the front inlet and the transfer lines being set to 150°C and 155°C respectively. The carrier gas used was helium and it was set to a pressure of 15 PSI.

The MSD was set to single ion mode (SIM) in order to detect the following analytes:

| **Analyte** | **Mass** |
|---|---|
| **Water** | 18 |
| **Nitrogen** | 28 |
| **Oxygen** | 32 |
| **Argon** | 40 |
| **Carbon Dioxide** | 44 |
| **Chlorine** | 70 |
| **Ozone** | 48 |

The ionization source temperature was set to 230°C and the quadrupole temperature was set to 150°C. The electron energy was set to 15 V.

Mass spectrometry data was obtained from analysis of the gas phase headspace of the water, the composition, and hypochlorite solution. The raw area counts obtained from the mass spectrometer were normalized to the area counts of nitrogen in order to eliminate any systematic instrument variation. Both nitrogen and water were used as standards because they were present in equal volumes in the vial with nitrogen occupying the headspace and water being the solvent. It was assumed that the overall volume of water and nitrogen would be the same for each sample after degassing. In order for this assumption to be correct, the ratio of nitrogen to water should be the same for each sample. A cutoff value for the percent relative standard deviation (% RSD) of 5% was used. Across all nine samples, a% RSD of 4.2 was observed. Of note, sample NaClO⁻³ appears to be an outlier, thus, when removed, the % RSD drops to 3.4%.

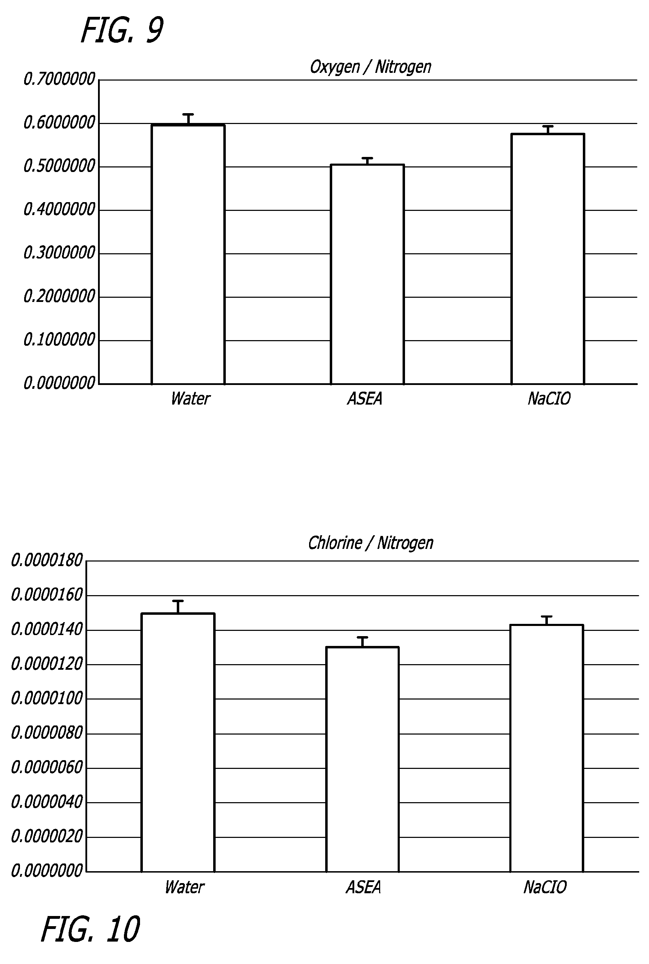

Figures 9-11 illustrate oxygen/nitrogen, chlorine/nitrogen, and ozone/nitrogen ratios. It appears that there were less of these gases released from the composition than from either water or nitrogen. It should be noted that the signals for both ozone and chlorine were very weak. Thus, there is a possibility that these signals may be due to instrument noise and not from the target analytes.

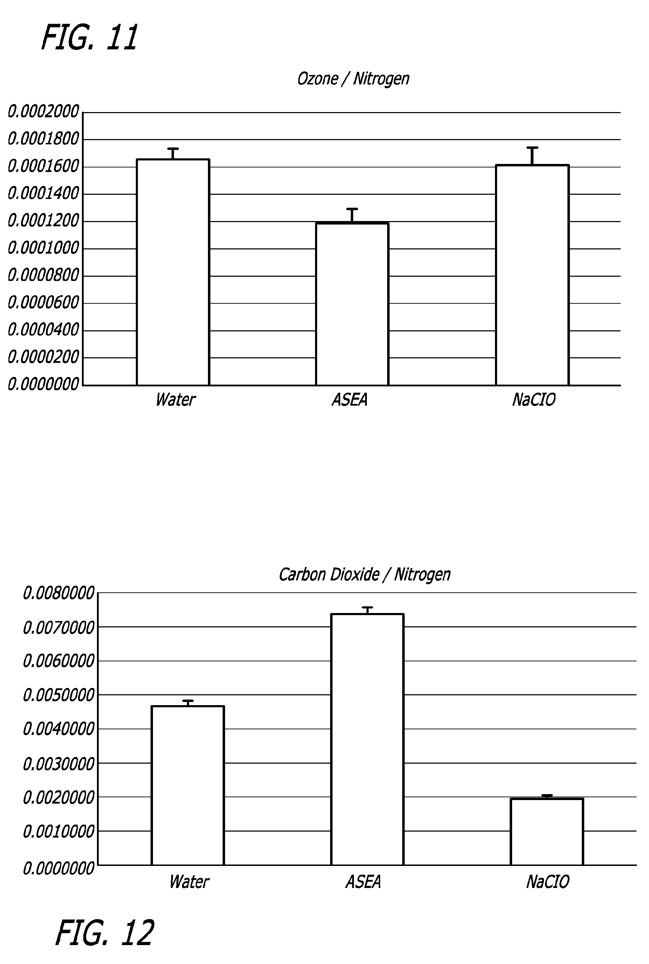

Figure 12 illustrates the carbon dioxide to nitrogen ratio. It appears that there may have been more carbon dioxide released from the composition than oxygen. However, it is possible that this may be due to background contamination from the atmosphere.

Based on the above, more oxygen was released from both water and sodium hypochlorite than the composition.

### EPR

Two different composition samples were prepared for EPR analysis. The composition with nothing added was one sample. The other sample was prepared by adding 31 mg of DIPPMPO to 20 mL of the composition (5.9mM), vortexing, and placing the sample in a 4°C refrigerator overnight. Both samples were placed in a small capillary tube which was then inserted into a normal 5 mm EPR tube for analysis.

EPR experiments were performed on a Bruker EMX 10/12 EPR spectrometer. EPR experiments were performed at 9.8 GHz with a centerfield position of 3500 Gauss and a sweepwidth of 100 Gauss. A 20 mW energy pulse was used with modulation frequency of 100 kHz and modulation amplitude of 1G. Experiments used 100 scans. All experiments were performed at room temperature.

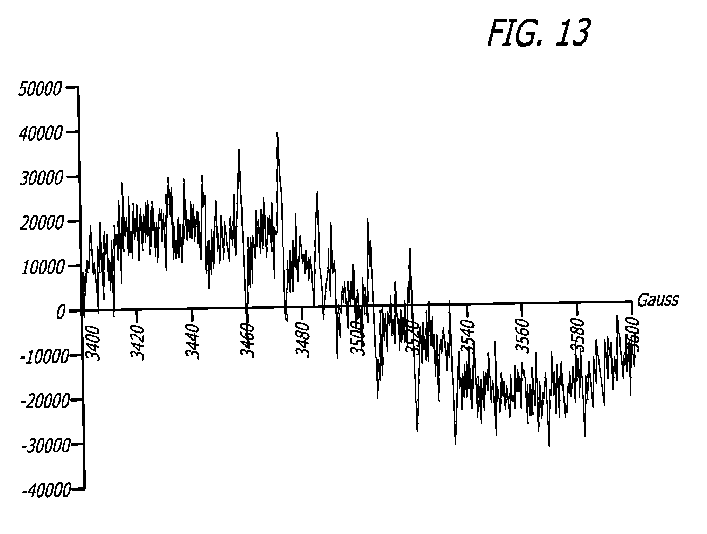

EPR analysis was performed on the composition with and without DIPPMPO mixed into the solution. Figure 9 shows the EPR spectrum generated from DIPPMPO mixed with the composition. The composition alone showed no EPR signal after 100 scans (not presented). Figure 13 illustrates an EPR splitting pattern for a free electron. This electron appears to be split by three different nuclei. The data indicate that this is a characteristic splitting pattern of OH• radical interacting with DMPO (similar to DIPPMPO). This pattern can be described by ¹⁴N splitting the peak into three equal peaks and ¹H three bonds away splitting that pattern into two equal triplets. If these splittings are the same, it leads to a quartet splitting where the two middle peaks are twice as large as the outer peaks. This pattern may be seen in Figure 13 twice, with the larger peaks at 3457 and 3471 for one quartet and 3504 and 3518 for the other quartet. In this case, the ¹⁴N splitting and the ¹H splitting are both roughly 14G, similar to an OH• radical attaching to DMPO. The two quartet patterns in Figure 13 are created by an additional splitting of 47G. This splitting is most likely from coupling to ³¹P, and similar patterns have been seen previously. The EPR spectrum in Figure 13 indicates that there is a DIPPMPO/OH• radical species in the solution.

### Example 3

### Delivery of Composition to Exercising Mice

Studies have shown that supplementation with green tea extract for 8-10 weeks in mice resulted in increased treadmill time to exhaustion compared to control mice. Higher muscle glycogen and increased fatty acid beta-oxidation were measured in exercised mice treated with green tea extract. Based on these studies, further exploration into other supplements that can increase physical properties such as time to exhaustion, VO₂ₘₐₓ. and the like may be useful.

The effect of composition (ASEA) ingestion on treadmill endurance capacity, fuel substrate utilization, tissue inflammation, and tissue oxidative stress in mice was studied. If ASEA causes increased fatty acid mobilization then endurance capacity can be improved in mice taking ASEA (compared to placebo). Sparing of muscle glycogen can be seen when taking ASEA. Mice were given the equivalent of about half the human ASEA dose.

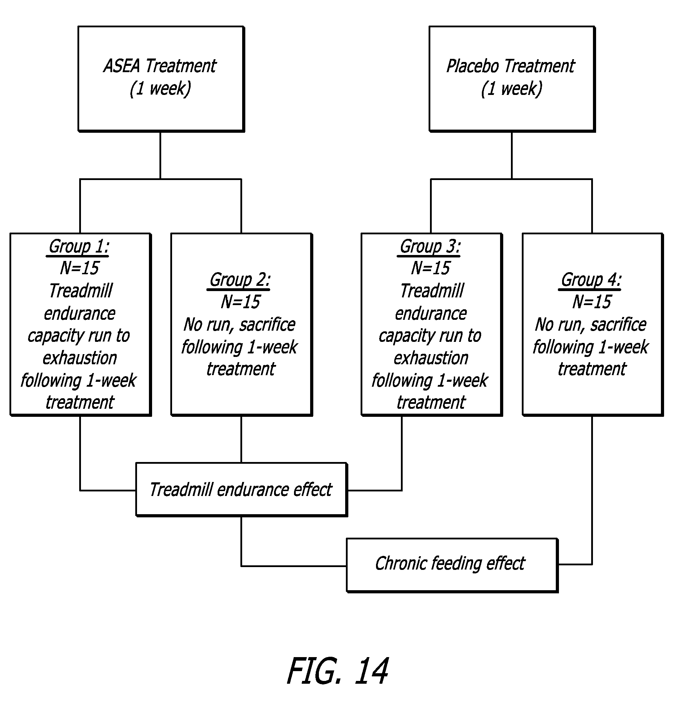

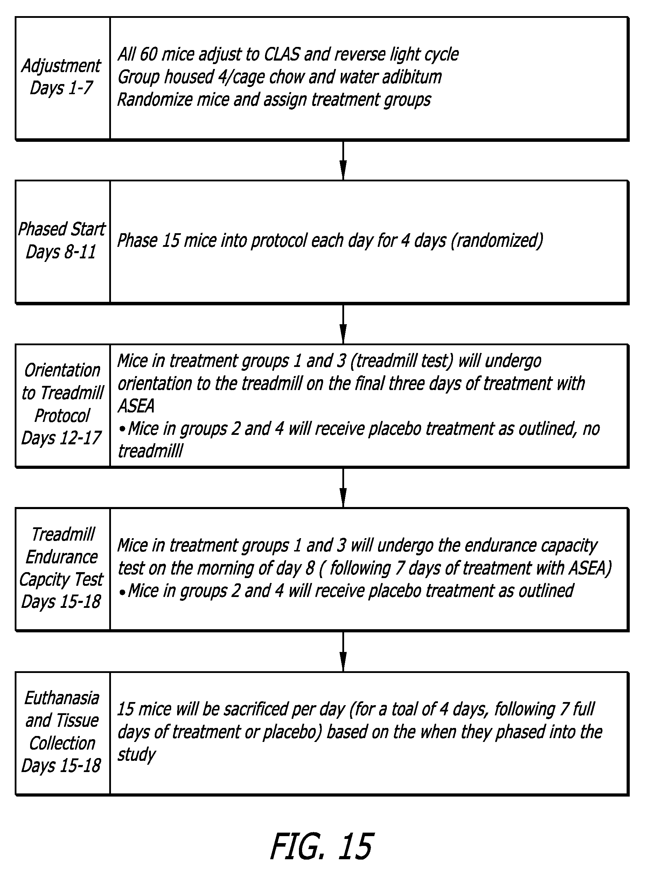

Six-month old male specific pathogen-free C57BL/6 laboratory mice (n=60) were purchased from Jackson Laboratory. Mice were randomly assigned to each of four treatment groups (n=15 each) as illustrated in Figure 14. A total overview of the mouse preparation and study is illustrated in Figure 15.

This particular strain and model of mouse has been used in previous studies involving both exercise and nutritional intervention studies. Thus, the use of this strain allowed comparison to data from other studies. Mice can be a suitable substitute for humans for this type of study because mice are genetically similar to humans and thus data obtained in this study will be translatable to human intervention studies.

All animal procedures took place in the Center for Laboratory Animal Sciences (CLAS) at the North Carolina Research Campus and protocols were reviewed and approved by the Institutional Animal Care and Use Committee (IACUC).

ASEA or placebo (same ingredients as ASEA composition without the proprietary signaling molecules added) was administered to the mice via gavage once per day for 1-week. The average body mass of all the mice at the start of the study and the volume of ASEA used for the gavaging were determined, but the volume did not exceed 0.3 mL per mouse. Guidelines for gavage are as the follows: "the volume should not exceed 1- 2% of body weight (= 0.2-0.4 mL for a 20 g mouse)". Thus, a volume of 0.3 mL for a 6 month old 30g mouse is well below this volume suggestion.

The composition was not palatable and the mice did not drink it willingly. Gavage was an acceptable alternative to ensure the mice did not become dehydrated simply because they would not drink the study composition. The gavaging was performed by the animal husbandry staff at CLAS. In one embodiment, mice can be given an amount of the composition that is equivalent to a daily human dose as described herein.

Following the 1-week (7 days) treatment period mice were euthanized and tissues harvested for further analysis of outcome measures. The four groups of mice were phased into the 1-week protocol each day. For example, if Group 1 started the protocol on a given day, Group 2 would begin the protocol on the following day, Group 3 would be begin the following day, and Group 4 the day after that. Mice from Group 1 would then be euthanized following the final treadmill test (7th day of treatment), Group 2, Group 3, and Group 4 each on subsequent days. Thus, total time for the mouse protocol was 11 days. There was overlap of orientation treadmill days, with maximal treadmill testing and euthanasia days. As stated, prior to euthanasia, mice from Group 1 and Group 3 underwent an endurance treadmill test to exhaustion using the protocol summarized in the following Table.

| **Time (min)** | **Speed (m/min)** | **Notes** |
|---|---|---|
| 1 | 0 | Adjustment to treadmill |
| 5 | 10 | "warm up" period |
| 2 | 12 | |
| 2 | 14 | |
| 2 | 16 | |
| 2 | 18 | |
| 2 | 20 | |
| 2 | 22 | Speeds between 20-24 m/min correspond to roughly 80% VO₂ₘₐₓ for mice |
| 2 to end | 24 | Mice stay at this speed until they reach exhaustion (e.g., sit on shock grid for 5 full seconds) |

During the three day period preceding the maximal endurance test, mice were oriented (trained) to the treadmill for 15 min/day. Speeds for the training days were about 10 m/min, 15 m/min, and 18 m/min respectively. Then, on the final day of treatment mice underwent a maximal endurance capacity test on the treadmill.

Mice from Group 2 and Group 4 were not submitted to an endurance capacity test and were euthanized at the end of 1-week treatment. Tissues harvested from these mice were collected to assess the chronic effects of the test composition in absence of an exercise intervention. All blood/plasma and tissues were snap-frozen in liquid nitrogen and stored at - 80°C until assayed.

For the treadmill orientation and endurance protocols, mice were run on a multilane rodent treadmill (Columbus Instruments, Columbus OH) equipped with a shock grid at the back. Once each mouse was placed in a treadmill lane, a 1 minute resting period was initiated. At this point, the mouse was able to adjust to the inside of the treadmill chamber. Following the 1 minute rest period, the treadmill belt was started at a speed of about 10 m/min, and the protocol described in the above Table was followed.

Mice were allowed to run until they were no longer able to keep up with the belt and the hind limbs stayed on the shock grid for more than about 5 seconds. When the mouse was no longer running (as assessed by sitting on the shock grid with all 4 paws off of the belt for more than 5 seconds), the mouse was removed from the shock grid immediately and placed back into the home cage. The mice were then monitored for recovery for a period of at least about 20 minutes following the orientation bouts.

The maximal endurance test occurred only once per mouse, and mice were euthanized immediately following the test. The test ended when the mouse could not run off the shock grid onto the treadmill at any point during the test or if signs of exhaustion (signs of above normal heart rate and ventilation) were evident.

The signs of exhaustion used included a mouse sitting on the shock grid for more than 5 seconds, rapid breathing, and/or increased heart rate. It has been our experience that mice that are not fatigued do not show these signs and will continue to run within 5 seconds of stopping. These procedures follow national recommendations (American Physiological Society's, Resource Book for the Design of Animal Exercise Protocols, 2006) based on research in the area. If at any point during the test a mouse got its foot caught between the shock grid and the treadmill the test was immediately terminated. If the mouse was injured and needed treatment, proper procedures were followed and vivarium staff was notified. If the mouse was deemed not injured, it was allowed to recover and placed back in its home cage and re-tested the following day. Once the mouse completed the protocol the mouse was placed back into its home cage. Generally, mice are usually back up and jumping around the cage within 30 seconds of re-exposure to the home cage following an endurance test. However, mice were still monitored several times during the 20-60 minutes following the procedure and notes were taken of any abnormalities such as apathy or decreased food consumption.

Some form of motivation was needed to make the mice run on the treadmill, particularly in the orientation sessions. A variety of forms of motivation can be used. The three most common techniques are, use of shock grid, use of air puffs, and manually tapping a mouse's tail. Use of air puffs have the potential to be ineffective and possibly confounding to data analysis. Given the standard rodent treadmill that is used in this type of testing that encloses the treadmill, manually tapping the tail was not ideal. Thus, shock grids were the best method of motivation for exercise on the treadmill.

The shock grid was positioned at the back of the treadmill. The shock grid delivered pulsed shock at an average current of 1.0 milliamperes at 150 volts (the shock grid was adjustable within a range of 0-3.4 mA). The shock grid was regularly checked with an ampmeter to ensure proper functioning. The shock levels used were 22 times less than that accepted in the literature. Also, the amperage of the system was 167-500 times less than lethal levels for mice, and the total power of the system was 60 times less than lethal levels for mice. No new data or guidelines existed to suggest that the use of a shock grid with our proposed settings was anything but appropriate.

Based on a similar study the effect size was calculated to be 1.647. Using p=0.0125 for significance during a priori power analysis. Using G-Power the following calculation was made and a least significant number of animals is assumed to 12/group. 15 animals per group were used (with estimated power of 0.95) to account for any loss of power if any animals do not make it through the protocol.

**Analysis: A priori: Compute required sample size**

| Input | Tail(s) | Two |
|---|---|---|
| | Effect size d | 1.6470588 |
| | a err prob | 0.0125 |
| | Power (1-β err prob) | 0.95 |
| | Allocation ratio N2/N1 | 1 |

| Output | Noncentrality parameter δ | 4.5106563 |
|---|---|---|
| | Critical t | 2.6694793 |
| | Df | 28 |
| | Sample size | 15 |
| | Actual power | 0.9604227 |

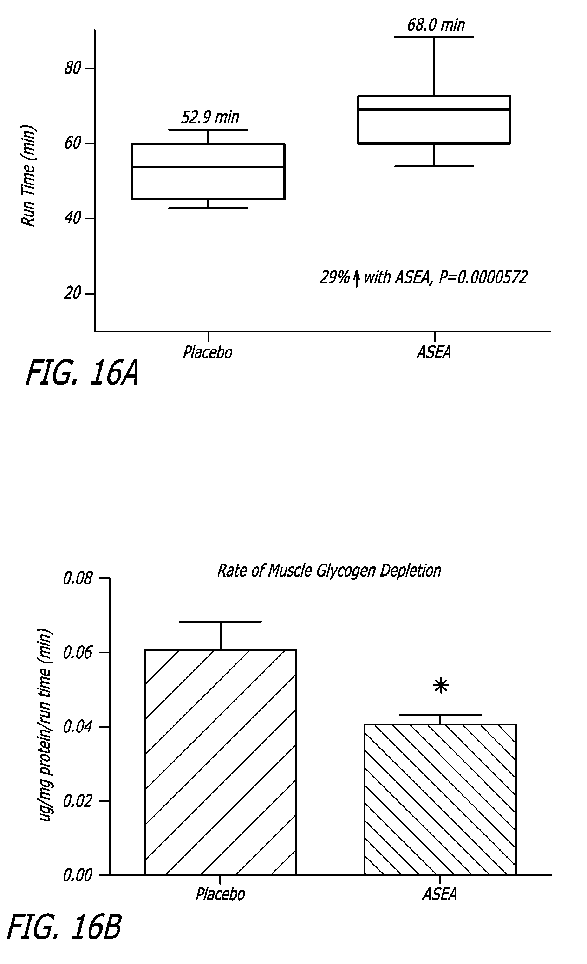

Based on the results from the mouse study, results are illustrated in Figures 16-20. Figure 16 (A and B) illustrates that mice who were administered ASEA had an increased run time to exhaustion. As such, ASEA can be used to increase time to exhaustion in athletes when exercising.

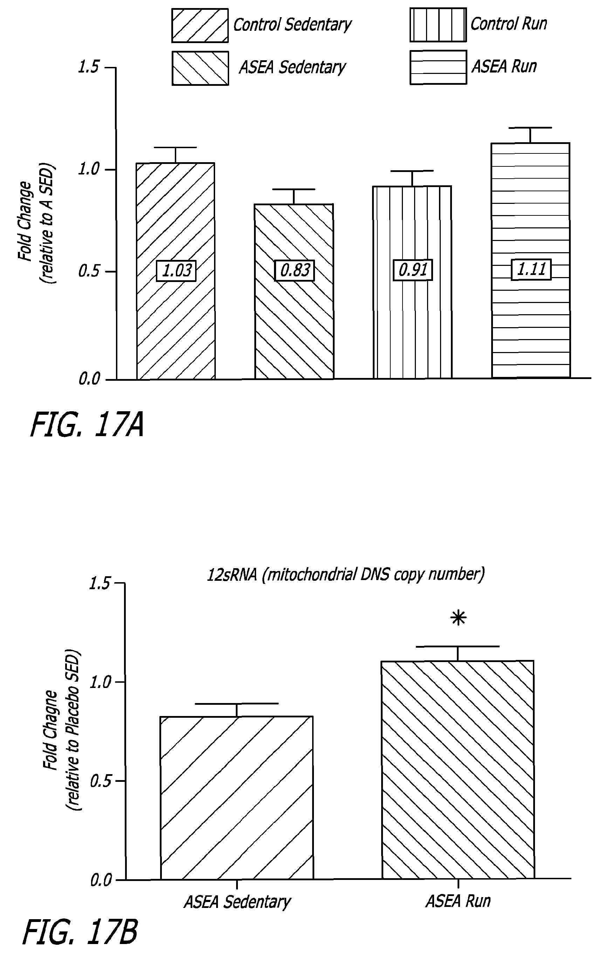

Figures 17A and 17B illustrate the fold change relate to ASEA of different mouse groups; P=0.042. This measurement tracks 12sRNA (mitochondrial DNA copy number). One week ASEA consumption in sedentary mice did not increase muscle mitochondria density. An interaction between one long endurance exercise bout to exhaustion was observed with ASEA vs. ASEA sedentary (P<0.05). Fold change increased when ASEA was delivered along with exercise, but fell when exercise was not present. This supports that ASEA helped decrease the level of oxidative stress in the muscle.

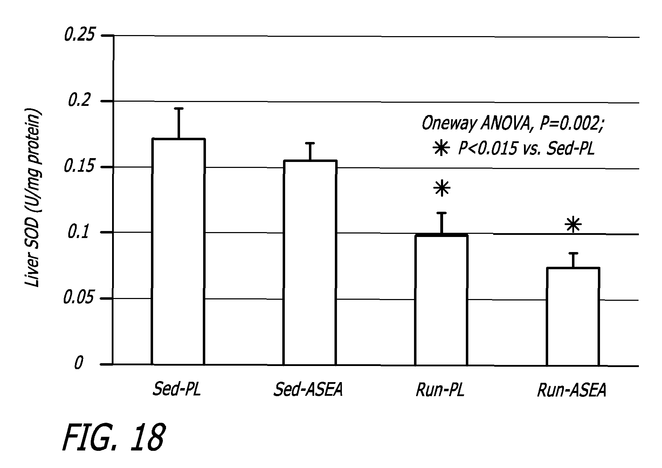

Figure 18 illustrates that SOD produced in the liver decreases in mice when administered ASEA and subjected to exercise. U is the amount of enyzme needed to inhibit 50% dismutation of the superoxide radical. An acute bout of exercise activates CuZnSOD activity, but most studies reported no change in its mRNA and enzyme protein levels, suggesting that the increased activity was due to increased O²⁻ concentration. This result can indicate that ASEA linked to exercise can reduce oxidative stress.

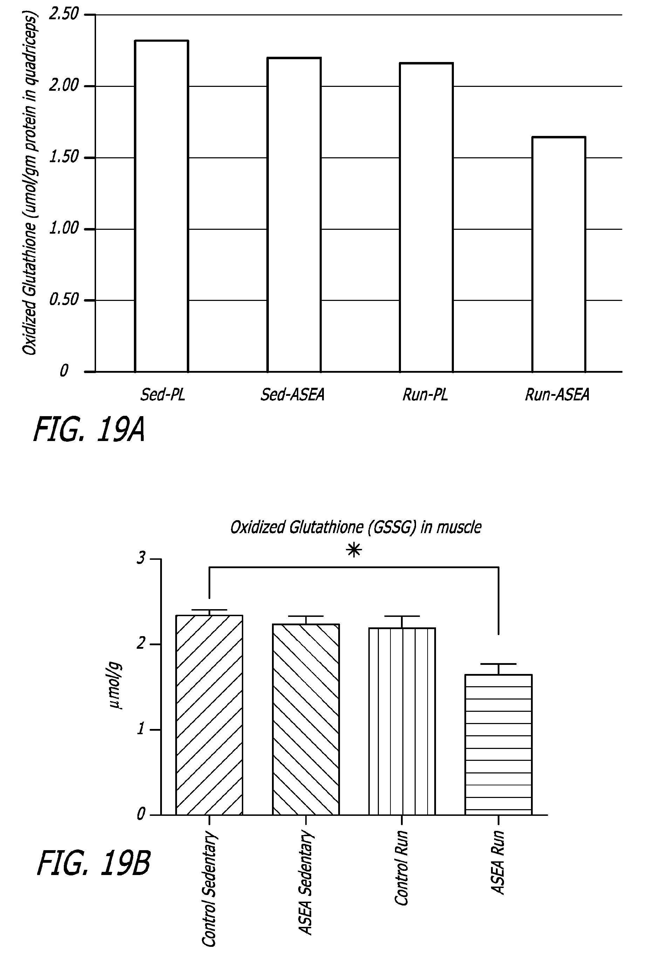

Figures 19A and 19B illustrate that oxidized glutothione decreases in mice when administered ASEA and subjected to exercise. This result can indicate that ASEA linked to exercise can reduce oxidative stress.

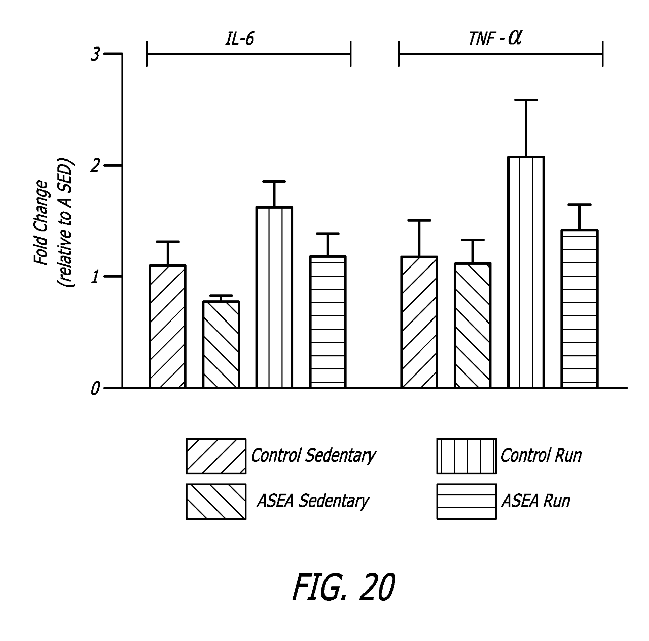

Figure 20 illustrates that exercise increased mRNA (gene expression) for IL-6 and TNF-alpha, indicating the typical pro-inflammatory response. ASEA tended to reduce gene expression for these inflammatory cytokines.

### Example 4

### Human Biking Exercise Study

A study was performed to estimate the increase in metabolism of individuals using the present systems and methods wherein the subjects drank an ASEA composition(s). The study was performed at the Metabolomics Laboratory, North Carolina Research Campus, David H. Murdock Research Institute and Appalachian State University. A goal of the study was to measure the influence of ASEA on small molecules (metabolites) that can shift in response to supplementation. The shift in metabolites, depending on the nutritional product, may represent effects on inflammation, oxidative stress, and physiologic stress.

Twenty-two subjects participated in the study. Each subject was tested for baseline values of VO2max and body composition. Then, ten participants were given an ASEA composition once a day for seven days and ten subjects were given a placebo once a day for seven days.

On the day of the first phase of the study, blood and urine were collected from all twenty-two participants and then each of the twenty-two participants biked 75 km. Blood and urine were collected just prior to finishing the 75 km biking and one hour thereafter. Results are tabulated below.

A washout period of three weeks then lapsed throughout which participants did not entertain an ASEA composition. After the three weeks, the participants crossed-over and were given the opposite composition for 7 days. The same routine was again performed (blood urine, 75 km biking, blood urine, blood urine one hour post). Data is tabulated below.

Athletes ingesting ASEA for seven days started the 75 km cycling trial with high blood free fatty acids leading to increased fat oxidation and a sparing of amino acids (and potentially muscle glycogen).

### Example 5

### Human Running Performance

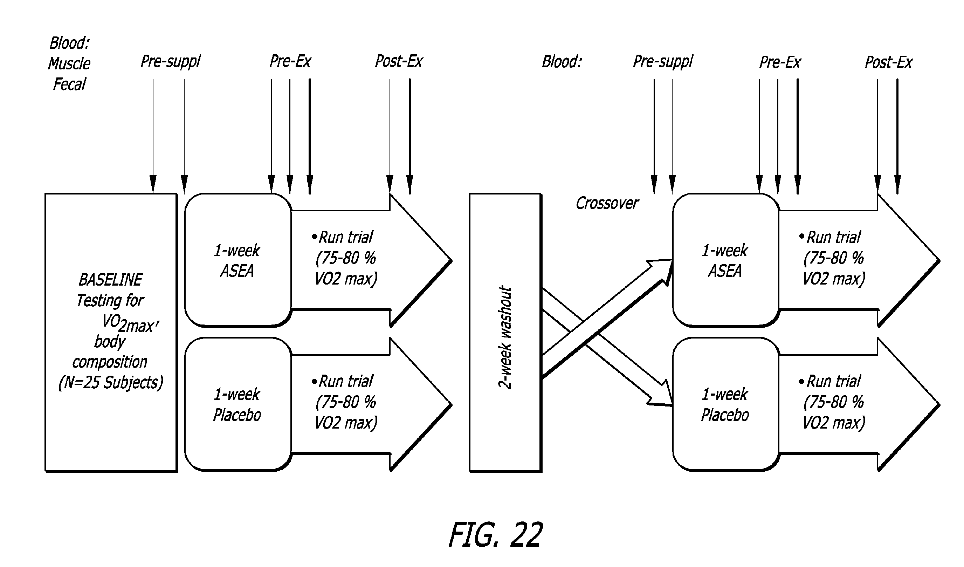

A study to determine if a composition of the invention versus placebo ingestion during a 2-week period improves run time to exhaustion when athletes run on treadmills with the speed adjusted to 70% VO₂ₘₐₓ· A flow chart of the study protocol is illustrated in Figure 22.

Blood and skeletal muscle biopsy samples are collected and analyzed for shifts in metabolites and glycogen utilization, respectively, to study underlying mechanisms.

Metabolites and glycogen utilization are altered when a composition of the invention is used alongside exercise.

### Example 6

### Efficacy of Ingesting ASEA on Disease Risk Factor Change in Overweight/Obese Women

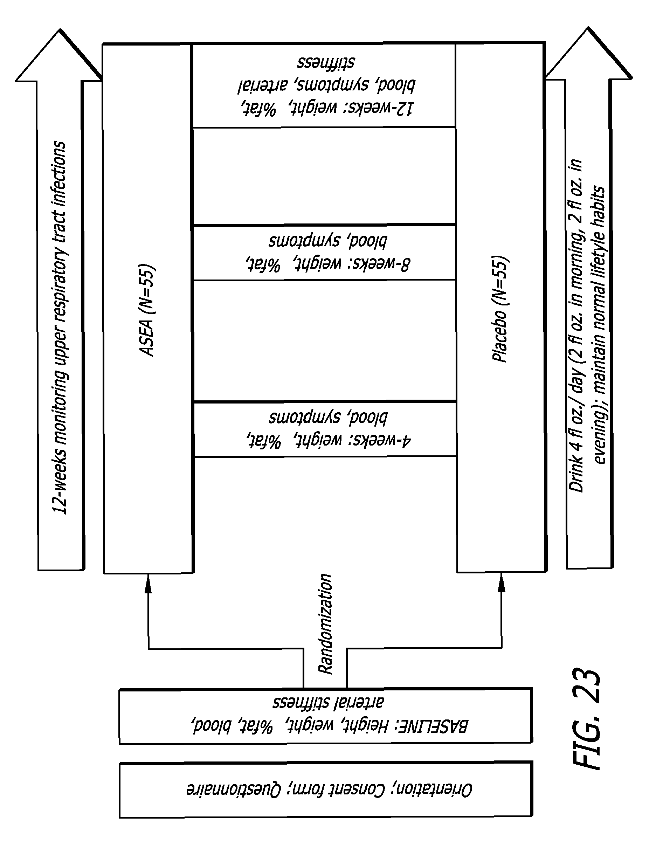

A 12-Week, randomized trial is performed accord to the protocol in Figure 23. The study evaluates the effectiveness of 4 fl oz/day ASEA compared to placebo over a 12-week period in helping adult women improve disease risk factors associated with arterial stiffness, inflammation, cholesterol status, blood pressure, oxidative stress and capacity, fasting serum glucose, and metabolic hormones.

Ingestion of ASEA over a 12 week period decreases arterial stiffness, decreases inflammation, improves cholesterol status, decreases blood pressure, decreases oxidative stress and capacity, decreases fasting serum glucose, and alters metabolic hormones.

### Example 7

### Effect of an Immune-Supporting Supplement. ASEA. on Athletic Performance

Described is a pilot study used to measure the possible effects of an immunesupporting supplement on athletic performance as measured by a standard VO₂ₘₐₓ and Ventilatory Threshold (VT) athletic endurance test.

The objectives of the pilot study were to (1) confirm the general observation that an immune-supporting supplement has an effect on athletic performance and (2) determine the specific physiological parameters: Heart Rates (HR), volume of O₂ inspired (VO₂), volume of CO₂ expired (VCO₂), volume of expired gas (VE), Respiration Rate (RR), Respiratory Exchange Ratio (RER), Aerobic Threshold (AeT), Anaerobic Threshold (AT), VO₂ₘₐₓ and Ventilatory Threshold (VT) that are affected by oral ingestion of this supplement during both the aerobic and anaerobic phases of exercise.

The immune-supporting supplement, a composition of the invention, contains a balanced mixture of Redox Signaling molecules that purportedly increases the efficiency of the communication channels between cells, enabling faster response of the immune system and cellular healing activities. Enzymes in the body also break down these Redox Signaling molecules into salt water and nascent oxygen. There are two proposed mechanisms involving Redox Signaling that can affect athletic performance, (1) increased efficiencies in cellular absorption or use of oxygen, prolonging aerobic metabolism, and (2) more efficient processing of lactate energy stores and tissue repair mechanisms, prolonging anaerobic metabolism.

During physical activity, the increased power requirements from muscle tissues require increased metabolism of available energy stores. Sustainable aerobic metabolism of sugars can supply this energy demand as long as there is an adequate supply of oxygen and sugars in the blood. As energy demands exceed the ability of the respiratory and cardiovascular system to deliver sufficient oxygen to the muscle tissue, methods involving the anaerobic metabolism of carbohydrates, creatines, pyruvates, etc. start to become prevalent.

Anaerobic metabolism supplies the excessive demand for energy but is accompanied by the production of CO₂ and lactates. Prolonged or excessive anaerobic metabolism depletes the available energy stores faster than they can be renewed; the buildup of CO₂ and lactates can also interfere with aerobic metabolism and thus, when the energy stores are spent, exhaustion will result.

Because anaerobic metabolism is marked by an excess in CO₂ and lactate production, it can be monitored by measuring the excess CO₂ exhaled during exercise or the buildup of lactates in the blood. The Ventilatory Threshold (VT) is the point where the excess CO₂ is first detected in the expired breath; it is related to the point at which anaerobic metabolism is starting to become prevalent.

In this pilot study, VT was determined graphically from the VCO₂ vs. VO₂ graph. VCO₂ is the volume of CO₂ expired per minute and VO₂ is the volume of O₂ inspired per minute. VO₂ₘₐₓ is simply the maximum volume of O₂ inspired per minute possible for any given individual. VO₂ₘₐₓ is measured in mL/kg/min (milliliters of O₂ per kilogram of body weight per minute). VO₂ₘₐₓ is measured at the peak of the VO₂ curve. The Aerobic Threshold (AeT) was determined by the software and indicates when fat-burning metabolic activities start to be dominated by aerobic metabolism. The Anaerobic Threshold (AT) was also software-determined and marks the point where the anaerobic metabolism starts to completely dominate.

Recruitment Methods: A standard VO₂ₘₐₓ test was run on 18 athletes who responded to recruitment flyers posted in athletic clubs and to invitations extended to a local competitive Triathlon team. The participants were selected based on answers from qualification questionnaire which affirmed that they:
1. Perform a rigorous physical workout at least five hours per week on average.
2. Have no medical conditions that might prevent participation
3. Agree to follow diet and hydration instructions.
4. Will perform only normal daily routines during the study.
5. Have no history of heart problems in the family.

The final selections were athletes of a caliber much higher that the expectations reflected in the recruitment flyers, a majority being athletes involved in regular athletic competitions. All of the participants had never taken the supplement prior to the study.

The participants did not receive any monetary compensation, but did receive a case of product and results from the VO₂ₘₐₓ tests.

The VO₂ₘₐₓ testing was done at an athletic club by accredited professionals holding degrees in exercise physiology and with more than 10 years daily experience in administering VO₂ tests. The participants were given a choice of performing the test on either a treadmill or a stationary cycle. A CARDIOCOACH® metabolic cart measured heart rate (HR), inspired and expired gases (VO₂, VCO₂, VE) and recorded weight, height, age, and body mass indexes (BMI). Power settings on the treadmill or cycle were recorded every minute.

Each participant was scheduled to take two VO₂ₘₐₓ tests, (1) a baseline test and (2) a final test. The baseline test was performed before any supplement ingestion. The participants drank 4 oz. of the supplement per day between the baseline test and the final test (7 to 10 days later) and drank 8 oz. of the supplement ten minutes before starting the final test. For the baseline test, the power settings on the cycle or treadmill were determined by the test administrator. The power settings for the final test were matched exactly to the power settings of the baseline test for each participant. Participants were encouraged to strictly maintain their regular diet and exercise routine and to come to each test well hydrated (at least 8 oz. of water in the last 2 hours before each test).

Each participant was fitted with a breathing mask and heart monitor. Each VO₂ₘₐₓ test consisted of a 10 minute warm up period where participants walked or cycled at a low power setting determined by the administrator. This was followed by a ramp up period, where the administrators increased the power settings every minute, according to their evaluation of the physical condition of the participant, and termination when the administrators started seeing the indications of a maximum VO₂ reading when RER (VCO₂/VO₂) > 1.0 or at the administrator's discretion. The administrators had ample experience in obtaining consistent VO₂ₘₐₓ results on this equipment, estimated at about 6% test to test variation over the last 5 years.

The raw data (HR, VO₂, VCO₂, VE, Power Settings) were collected from the CARDIOCOACH® software for analysis. Data points were automatically averaged over 15 to 25 second breath intervals by the software, VO₂ₘₐₓ is also determined by the software with an averaged VO₂ peak method. VT was determined graphically from the slope of the VCO₂ vs. VO₂ graph.

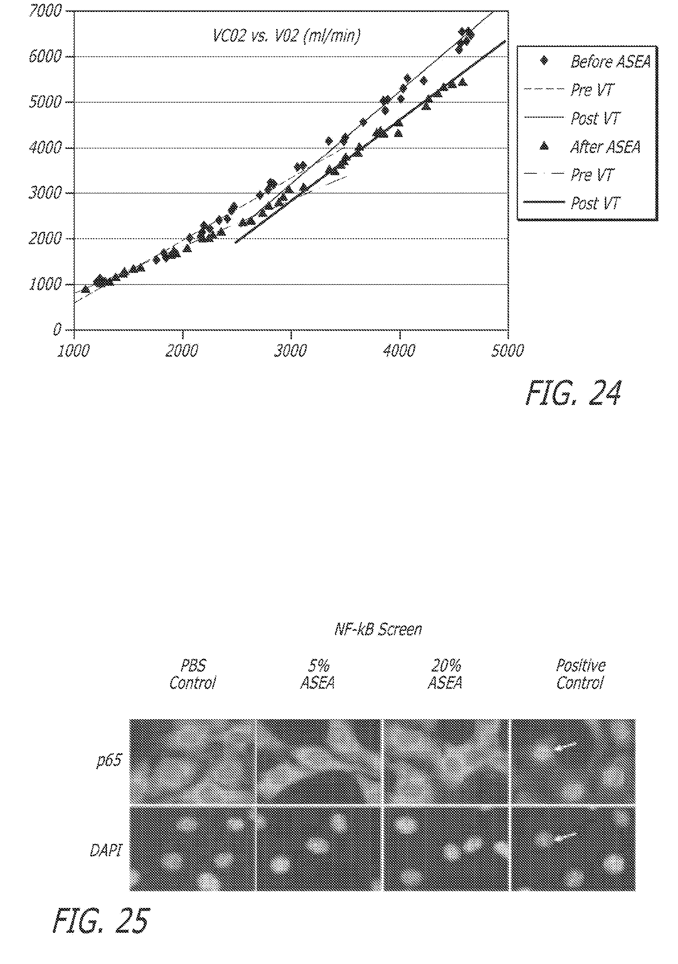

Linear regression methods were used to determine the slope, change in VCO₂ over change in VO₂. In theory, when aerobic metabolism switches to anaerobic metabolism, the volume of CO₂ expelled (VCO₂) is increased in proportion to the Volume of O₂ inhaled (VO₂). This is reflected as an increase of slope on the VCO₂ vs. VO₂ graph, seen as a clear kink on the graph around the VT point. Linear regression was used to determine the slope both before VT and after. Slopes were determined by linear regression on the linear region of data points before and after VT point, excluding points surrounding the VT and near VO₂ₘₐₓ. The intersection of the before and after lines was used to determine the reported VT point (Figure 24).

Methods for determining the VT point on any individual participant were kept consistent from the baseline test to the final test. Average HR was averaged over the linear range of HR increase during the power ramp, excluding points a few minutes into the beginning and before the end of the data set. In every case, the same data analysis methods were used for the final test as were used for the baseline test for each participant.

Compliance to protocol was very high by both participants and administrators, based on answers to compliance questions. One data set was discarded for low VCO₂ values, probably due to a loose mask. The ventilatory data for this one participant was rejected, leaving 17 valid data ventilatory data sets. The Heart Rates (HR), however, were compared for all 18 participants.

| Total Participants | Average Age | Male/ Female | Average Weight (Kg) | Cycle/ Treadmill | Average BMI | Data Sets Selected |
|---|---|---|---|---|---|---|
| 18 | 41±9 | 16/2 | 76±11 | 7/11 | 24.4±3.4 | 17 |

The average VO₂ₘₐₓ reading over all participants (N=17) was measured at the relatively high value of 62.5 mL/kg/min, indicative of the quality of athletes in the sample. Only four participants had VO₂ₘₐₓ readings below 55 mL/kg/min; these four were not involved in competitive training programs.

The data shows that two significant changes in physiological parameters could be attributed to ingestion of the composition, as determined by a statistical paired t-test analysis. The average time taken to arrive at VO₂ₘₐₓ was increased by 10% with very high confidence (P=0.006) and the average time taken to arrive at Ventilatory Threshold (VT) was increased by 12% with a marginal level of confidence (P=0.08).

Given that the power ramp-up-points between the baseline and final test for each participant were identical, an increase in the amount of time to obtain VO₂ₘₐₓ and VT on the final test also indicates a higher average power outputs at such thresholds. Calibrated power output measurements were not available. However, the test administrator for the final test, upon reaching the maximum power recorded for the baseline test, regularly surpassed this maximum power before the participant reached VO₂ₘₐₓ on the final test.

All other physiological parameters (V_{O2max}, VT, AeT, AT, Start HR, HR at AeT, HR at AT, HR at VO₂ₘₐₓ. and overall average HR) were not significantly changed by supplement ingestion. The high level of consistency between the baseline and final test for these parameters, however, supports the repeatability of the tests. The test to test repeatability has an estimated standard deviation of less than 5% for all parameters.

| Averages (N=17) | Baseline | Final | Change | %Change | P-Value |
|---|---|---|---|---|---|
| VO₂ₘₐₓ (mL/kg/min) | 62.5 | 63.6 | +1.1 | +2% | - |
| VT (mL/kg/min) | 36.4 | 38.7 | +2.3 | +6% | 0.34 |
| Aerobic Thresh. (AeT) | 43.6 | 43.8 | +0.2 | +0% | - |
| Anaerobic Thresh. (AT) | 55.5 | 56.5 | +1.0 | +2% | - |
| Pre VT Slope of VCO₂/VO₂ | 1.030 | 1.030 | 0.0 | 0% | - |
| Post VT slope of VCO₂/VO₂ | 1.997 | 1.944 | -0.053 | -2.7% | - |
| Start Heart Rate (bpm) | 87.4 | 85.9 | -1.5 | -1% | - |
| Heart Rate at AeT | 147 | 145 | -2 | -2% | - |
| Heart Rate at AT | 165 | 165 | 0 | 0% | - |
| Heart Rate at VO₂ₘₐₓ | 174 | 175 | +1 | +1% | - |
| Heart Rate Overall | 137 | 134 | -3 | -2% | - |
| Time to VT (secs) | 306 | 344 | 38 | +12% | 0.08 |
| Time to VO₂ₘₐₓ(s) | 639 | 703 | 64 | +10% | 0.006 |

Of the 17 participants in the study, 70% of them experienced a significant increase in time to VO₂ₘₐₓ. 18% of the participants showing more than a 25% increase, 41% showing more than a 10% increase, 18% of the participants exhibiting no significant change and 12% showing a mild decrease (under 10%).

There was a moderate but significant correlation between the increases in "time to VO₂ₘₐₓ" and "time to VT" (correlation coefficient 0.35), meaning that an increase in time to reach VO₂ₘₐₓ was moderately but not always proportional to the increase in the time it took to get to VT. There is a strong correlation between increase in time to VO₂ₘₐₓ and decrease in the average overall heart rate (correlation coefficient -0.67), meaning that an increase in time to VO₂ₘₐₓ would most often be accompanied by a decrease in average overall heart rate.

Ingestion of the test supplement, a composition of the invention, for 7-10 days prior to and immediately before a VO₂ₘₐₓ test, was shown to significantly increase the time it took for 70% of the participants to reach VO₂ₘₐₓ under equivalent carefully regulated power ramp-up conditions. Time to VT likewise was significantly extended.

The extension of time to reach VT, under similar increasing demands for energy, is a direct indication that the aerobic phase of metabolism is being extended and/or the anaerobic phases somehow are being delayed as the demand for energy increases.

The lack of any other changes in the physiological parameters (V_{O2max}, VT, AeT, AT and associated heart rates) suggests that cardiovascular capacity, lung capacity and blood oxygen capacity and regulation were not affected. This assumption is reasonable, given that the short duration of this study excluded the possibility of training effects.

One feasible explanation for the results lies in the enhancement of aerobic efficiencies, meaning that more aerobic energy can be extracted at the same physiological state, or that the clearance of lactates or CO₂ becomes more efficient, again allowing greater aerobic efficiency. Note that "time to AeT" and "time to AT" were not compiled in this study, however changes in these parameters would be expected and might offer clues to determine the underlying mechanisms.

The results of this pilot test indicate that there is a strong case for athletic performance enhancement and further investigation is warranted. A placebo-based doubleblind test, measuring the more subtle effects in ventilation and heart rates along with increases in blood lactate levels during a controlled, calibrated power ramp would provide defensible evidence for this effect and better support for some specific underlying mechanisms of action.

### Example 8

### In Vitro Bioactivity Study

Described are a variety of results from *in vitro* experiments, performed at national research institutions, investigating the bioactivity of a composition disclosed herein, when placed in direct physical contact with living cells. Specific investigations include *in vitro* toxicity and antioxidant efficiencies of the master antioxidants glutathione peroxidase (GPx) and Superoxide Dismutase (SOD) inside living cells and the translocation of two well-studied transcription factors (NF-kB, NRF2) known to regulate toxic response and antioxidant production in human cells. Some preliminary work on concentration dependence was also done as well as cell proliferation, counts associated with induced oxidative stress in human cells.

The objectives of the investigations were (1) to determine if any signs of toxicity (NF-kB activation) are manifest when varying concentrations of a certain redox signaling compound, ASEA, are placed in physical contact with living cells, (2) to determine if such direct contact affects the antioxidant efficacy of glutathione peroxidase (GPx) and superoxide dismutase (SOD) and (3) to determine if such contact activates translocational transcription (NRF2) associated with increased expression of antioxidants in living human endothelial cells and to verify the expression of such transcription factors by Western Blot analysis, (4) to determine the effect of this redox signaling compound on proliferation cell counts of human cells and associated markers (LDH) for cell viability and health, (5) to determine the effects of this redox signaling compound on cells that were stressed with cytokines (Cachexin), radiation and serum starvation.

The immune-supporting composition contains a redox-balanced mixture of RXN [both reactive oxygen species (ROS) and reduced species (RS)] that are involved in a large variety of pathways and receptor-site activity in human cells. For example, when cells are damaged, for any reason (ex. toxins, DNA breaks or infections), the native redox signaling messengers inside the cells can become imbalanced, most often manifest by the accumulation of intracellular oxidants and ROS (oxidative stress). The cell, so affected, will activate defense and repair mechanisms aimed to restore proper redox-signaling homeostasis and proper cellular function. If repair efforts are unsuccessful and normal homeostatic redox balance is not able to be restored, then within a few hours, the excess oxidants and ROS in such cells will facilitate apoptotic processes to internally digest and destroy the dysfunctional cell. Healthy neighboring cells will then divide to replace it. A complete field of science called "redox signaling" has been founded to study such processes, with literally thousands of references available.

It is the nature of certain redox signaling molecules, when unbalanced or isolated, to elicit immediate recognizable toxic responses in exposed living cells; hydrogen peroxide is one example of such a redox signaling molecule. The first-line cellular response to toxic substances involves the translocation ofNF-kB into the nucleus as a precursor to the inflammatory response and other defense mechanisms. The movement ofNF-kB into the nucleus can be visibly tracked in a living cell under a fluorescence microscope with the aid of fluorescent tag molecules. The observation of nuclear translocation ofNF-kB is a sure marker that a toxic response has been initiated. Even low-level toxicity is detectable with this catch-all method; low-level concentrations of hydrogen peroxide, for example, produce an easily distinguishable positive toxic response.

A separate transcription factor, NRF2, moves into the nucleus in response to low-level oxidative stress and facilitates the increased production of antioxidants. Again, by the use of fluorescent tags, the nuclear translocation of NRF2 can be seen in cells under a fluorescence microscope. NRF2 nuclear translocation is a second-line-of-defense mechanism known to increase the production of protective enzymes and antioxidants such as glutathione peroxidase and superoxide dismutase. NRF2 translocation will often accompany low-level NF-kB activation and NF-kB activation (almost) always precedes NRF2 translocation. Substances that exhibit low-level toxicity, such as trace homeopathic toxins, have long been used to activate the NRF2 pathway in order to stimulate these natural defend-repair-replace mechanisms.

Enzymatic efficacy of antioxidants, such as Glutathione Peroxidase (GPx) and Superoxide Dismutase (SOD), can be determined through standardized ELISA tests that measure the time-related reduction of certain oxidants introduced into cell lysates after the living cells have been exposed to the test substance for a given period of time. The reagents of the ELISA test must be chosen as not to interfere or interact with the test substance. Other critical factors such as the time of exposure and concentration dependence must be experimentally determined.

Western Blot methods also exist to experimentally determine the quantities of GPx or SOD in cell lysates. These well-established molecular separation techniques and can be used to directly verify whether the quantity of such antioxidant enzymes has been increased in the sample. Measured antioxidant efficiency, however, remains the best indication of cellular antioxidant defense.

Monitoring cellular proliferation, cell counts and chemical indicators of cellular death are also commonly used to determine cellular viability and gross response to stressors such as radiation, cytokines and toxins. Cachexin, for example, is a potent toxin, a cytokine, that elicits immediate toxic responses and build-up of oxidative stress in exposed cells. Cells, so stressed, exhibit a greater tendency to undergo apoptosis and die, thereby releasing internal proteins (such as LDH) into the surrounding serum.

Normally, when the introduction of such stressors and toxins elicits oxidative stress conditions in the cell cultures, cell counts will fall, cellular proliferation will subside, and serum LDH levels will rise, indicating that cell death is occurring in the culture. Hydrogen peroxide, radiation and serum starvation can also elicit similar responses. Redox signaling messengers, as outlined above, are intimately involved in cellular reception of and response to such stressors; redox messengers are involved in mediating antioxidant production and action to protect the cells, repair mechanisms necessary to fix DNA and structural damage and also in mediating the apoptotic process that results in cell death.

Increasing the concentration of such redox messengers in the serum may serve to augment the efficiency of these normal cellular processes. The exact action of various redox signaling mixtures must be determined experimentally. Independent unpublished studies, involving Mass Spectroscopy, Florescent Spectroscopy and Electron Spin Resonance, have unmistakably verified the existence of several kinds redox signaling molecules in the composition described herein. Well-established redox electrochemistry also validates the existence of such redox signaling molecules. The stability of this redoxbalanced mixture is many orders of magnitude greater than expected. The confirmed preservation of unstable moieties in this supplement might be explained by the existence of certain stable molecular complexes, some of them verified by mass spectroscopy that can shield radical interactions. Intellectual property agreements, however, prevent the disclosure of the details.

The following research was conducted on a best efforts basis by a senior researcher at a national laboratory and is designed to assess basic mode-of-action when a composition of the invention is placed into direct contact with human cells:
1. The initial dose range projected for *in vitro* studies was extrapolated from a 10 mL of a composition of the invention /kg equivalent oral dose from human trials.
2. Glutathione peroxidase (GPx) and superoxide dismutase (SOD) ELISAs were used to determine whether a composition of the invention alters enzymatic activity in murine epidermal (JB6) cells.
3. LDH (non-specific cellular death) levels and cell proliferation rates were determined for various cell types exposed to a composition of the invention.
4. Human microvascular endothelial lung cells (HMVEC-L) were treated with a composition of the invention and cell lysates were analyzed by GSH-Px and SOD ELISAs to determine whether antioxidant enzyme activities are altered.
5. HMVEC-L cells were treated with a phosphate buffered saline solution (PBS)negative control, 5% and 20% concentrations of a composition of the invention and a Cachexin positive control to determine the nuclear translocation activity of the p65 subunit of NF-kB (cytokine transcription) at 30, 60, 90 and 120 min intervals. Fluorescent microscopy techniques were employed to image cellular response.
6. Step (4) was repeated except nuclear translocation activity of P-Jun was determined as an extension/verification of step 4.
7. Two cultures of HMVEC-L cells, one with normal random cell cycles and another with serum starvation were treated with low < 1 % concentrations of a composition of the invention to determine the nuclear activity of NRF2 (antioxidant transcription) at 30, 60, 90 and 120 minute intervals compared to a negative (PBS) control.
8. A Western Blot analysis was done on extra-nuclear and intra-nuclear fractions, separated by differential centrifugation, of serum starved HMVEC-L cell cultures exposed to < 1 % of a composition of the invention compared with a positive hydrogen peroxide control to determine phosphorylation events (oxidant action) in the extra-nuclear fraction and NRF2 (antioxidant transcription) in the intra-nuclear fraction at 0, 30, 60, 90 and 120 min intervals.
9. Normal random cell phases of HMVEC-L cells were exposed to radiation and then treated with a composition of the invention. Cell counts were taken to determine survival.
10. The efficacy of Cachexin reception in confluent-phase and normal-phase HMVEC-L cells was determined through changes in extracellular and intracellular LDH activity in cells exposed to various mixtures of Cachexin, PBS and a composition of the invention solutions.

Experimental Methods used to Assess Toxic Response in Primary Human Lung Microvascular Endothelial Cells (HMVEC-L): HMVEC-L cells (catalog# CC-2527) were purchased from Lonza (Walkersville, MD) as cryopreserved cells (Lot# 7F4273). Cells were thawed and maintained according to manufacturer's directions. Cell culture medium (proprietary formulation provided by Lonza) contained epidermal growth factor, hydrocortisone, GA-1000, fetal bovine serum, vasoactive endothelial growth factor, basic fibroblast growth factor, insulin growth factor-1 and ascorbic acid.

HMVEC-L Cell cultures in normal random cell cycles were exposed to highconcentration ASEA in the serum medium, concentrations of 5% and 20%, and analyzed in conjunction with cultures exposed to phosphate buffered saline solution (PBS) as non-toxic negative control and Cachexin (5 ng/mL) as a positive control (highly toxic). At intervals of 0, 30, 60, 90, and 120 minutes, aliquots of cells from each culture were placed under a fluorescent microscope, stained by fluorescent dyes designed to tag the p65 subunit of NFkB along with a DAPI fluorescent nuclear stain that aids the computer software to find the nuclei. Computer automated imaging techniques were used to determine the relative degree of translocation NF-kB into the nucleus via fluorescent analysis over several cells. As a reminder to the reader, P65 NF-kB translocation is the first-phase non-specific cellular response to toxicity. Thus the movement of the NF-kB into the nucleus, as seen visually in the microscope images, is a sensitive indicator of general toxic response.

Results of HMVEC-L Cells p65 subunit NF-kB screen for toxicity: Typical cell images are shown below for each culture. Translocation of p65 subunit of NF-kB into the nucleus was not seen in any cell cultures exposed to high-concentration a composition of the invention. Automated analysis confirmed this and indicated no toxic response at 0, 30, 90 and 120 minutes. In contrast, Cachexin exposed cells exhibited an immediate sustained toxic response (Figure 25).

Cachexin is positive control and induces the translocation of p65 subunit of NFkB from cytosol into nucleus. DAPI staining shows position of nuclei in these images (see white arrow). A composition of the invention (5 and 20% final v/v) did not induce nuclear translocation of NF-kB at 30, 60 and 120 min time points.

Given this null indication of toxicity after exposure to high concentrations of ASEA, another test was performed to confirm behavior.

Additional Method to Assess Toxic Response of HMVEC-L Cells (P-Jun): A similar methodology as that employed with NF-kB was employed to determine the nuclear translocation of an anti-phospho-Jun (AP-1 P-Jun) antibody index (P-Jun is another toxicityrelated redox-responsive transcription factor). HMVEC-L cells were again exposed to highconcentration ASEA. All procedures were similar to the NF-kB analysis except for the substitution of P-Jun fluorescent indicators and automated measurements taken over 100 cells in order to increase sensitivity. An additional naive (untouched) culture was also analyzed.

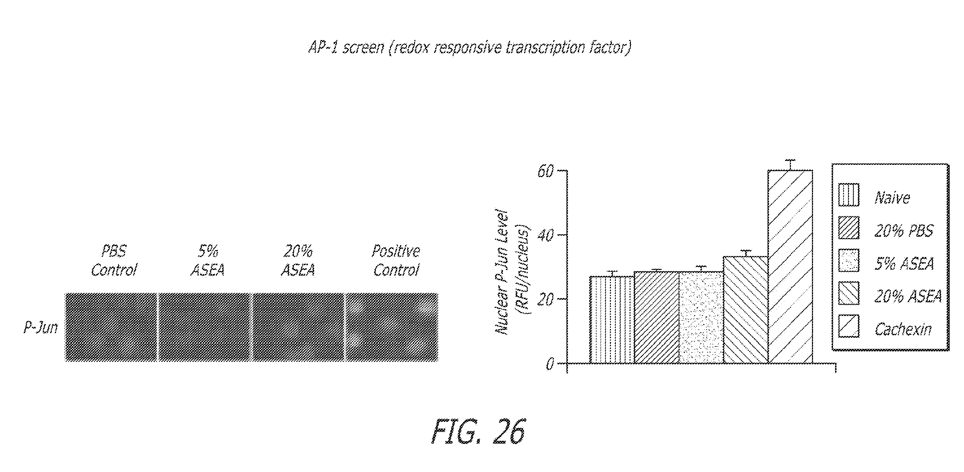

Results for P-Jun screen for toxicity (Figure 26): AP-1 index determined using anti-phospho-Jun (P-Jun) antibody. AP-1 is nuclear localized and upon activation, the phosphorylation status of P-Jun is increased. Anti-P-Jun antibody binds to the phosphorylated form reflected as an increase in fluorescence intensity (see Cachexin control). A consistent trend reflecting an increase in P-Jun levels was not observed for cells treated with 5% or 20% ASEA at 30, 60 and 120 min time points, while the Cachexin positive control significantly increased nuclear P-Jun levels at 30 min.

Again no toxic response was observed; there was no significant accumulation of P-Jun in the nuclei of cell cultures exposed to high concentrations of a composition of the invention. Automated analysis indicated no toxic response at 0, 30, 90 and 120 minutes, with a slight but non-significant increase for 20% a composition of the invention at the 30 minute time point; at other time points no increase was detected. In contrast, the Cachexin exposed cells (positive control), as expected exhibited an immediate sustained toxic response.

The results of the P-Jun analysis concurred with the response seen in the NFkB analysis. For both tests, there was no significant difference between a composition of the invention exposure and that of the negative PBS control for healthy random-phase HMVECL cells. This confirmed lack of toxicity was somewhat unexpected for this mixture of redox signaling molecules, considering that some of them, if isolated from the mixture, are known to elicit an immediate response.

Since nuclear translocation of NF-kB and P-Jun are typically the first responders to serum toxicity and are known to initiate the inflammatory response, especially in the ultrasensitive human endothelial cells, healthy human cells when directly exposed to a composition of the invention, are not expected to exhibit defensive behavior nor initiate inflammatory processes (such as the release of inflammatory cytokines). It is not certain from this data whether exposure would suppress or reverse the inflammatory process.

Blood serum levels of such redox signaling molecules, for all *in vivo* oral applications, would not exceed serum concentrations of 1 % and typically would be less than 0.1 %. Serum levels are expected to drop over time due to enzymatic breakdown of the components. Independent *in vivo* pharmacokinetic studies indicate that the active components in ASEA have approximately a 17 minute half-life in the blood and thus would be effectively cleared from the blood within a few hours. Thus no toxic response is expected due to exposure of healthy human cells at such levels. It has been seen in these *in vitro* studies that direct exposure of human cells to serum concentrations of up to 20% is still well tolerated. The complete lack of toxicity, comparable to the PBS control, is extremely rare and indicates that despite the reactivity of this mixture, it is well tolerated by human tissues and is native to or compatible with the extracellular environments.

Experimental Methods Used to Determine Antioxidant Efficacy of Glutathione Peroxidase (GPx): Cell cultures of standard murine epidermal cells (JB6) were exposed to various small concentrations of a composition of the invention (less than 1 %) and PBS solution for 24 hours. Cell lysates were prepared for measurements of GPx enzymatic activity using a commercially available ELISA kit (GPx activity kit, Cat #900-158) according to directions of the manufacturer (Assay Designs, Ann Arbor, MI). Decrease of oxidants due to GPx enzymatic activity was monitored over an 11 minute period of time after a chemical agent (cumene hydroperoxide) initiated the reaction. The decrease of oxidants is an indication of antioxidant efficacy. To determine GPx efficacy at various concentrations of PBS or a composition of the invention, three replications of oxidant residual in the samples were read every 2 min to generate the slope, indicating the decrease in relative fluorescence units (RFU)(oxidant residual) per minute.

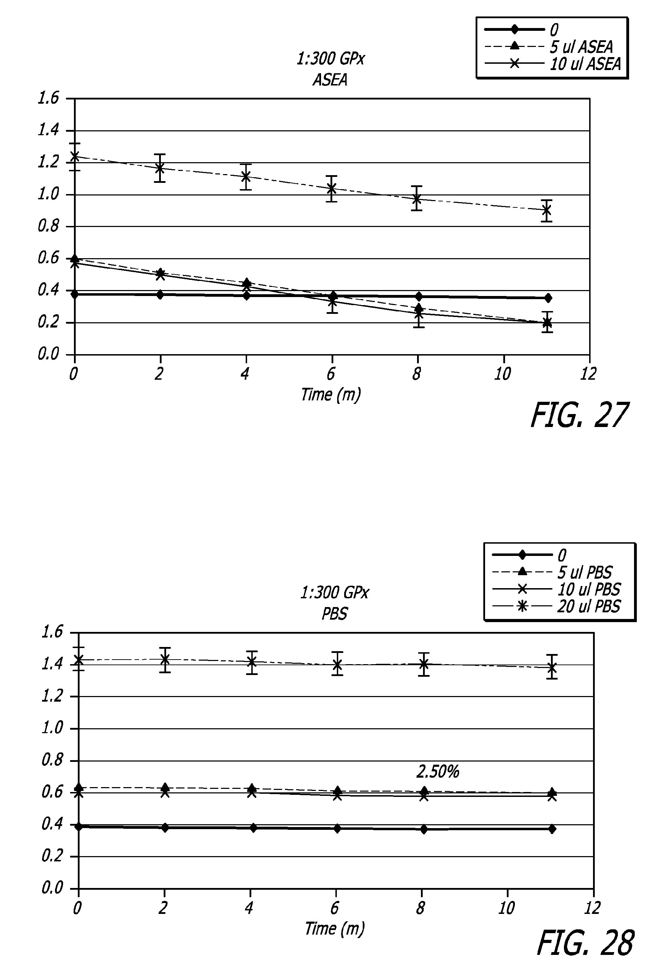

Results and Observations for GPx Antioxidant Efficacy Test: After activation, the reduction of oxidants over time was closely linear, as seen in the graphs below (RFU units on vertical scale). A well-defined slope was established over the 11 minute interval (Figure 27). Antioxidant activity is measured by reduction of oxidants over time (Figure 28).

A significant increase in antioxidant activity was seen in samples infused with ASEA compared to the PBS control (second graph).

Concentration dependency, however, was not seen between the 5ul, 10ul and 20ul infusions. This suggests that GPx antioxidant activity might saturate at concentrations lower than that represented by the 5ul infusion. Such considerations will be discussed later.

The table below summarizes the data shown on the preceding graphs.

| Sample Infusion Volume (< 1% total volume) | Slope for PBS Control (% reduction/minute) | Slope for ASEA (% reduction/minute) |
|---|---|---|
| 0 ul | 0.1% | 0.1% |
| 5 ul | 0.1% | 3.6% |
| 10 ul | 0.2% | 3.6% |
| 20 ul | 0.3% | 3.7% |

The raw data reflects more than a 10 fold increase in antioxidant activity related to ASEA infusion. Taking into account experimental uncertainties, it is 98% certain that the serum infusion of small concentrations (< 1%) of a composition of the invention increased antioxidant efficiencies by at least 800%. Further investigations should be done to confirm this increase and explore concentration dependence for these low-level serum concentrations.

Experimental Methods Used to Determine Antioxidant Efficacy of Superoxide Dismutase (SOD): Human HMVEC-L cells were treated with 10% phosphate buffered saline (PBS; vehicle control), 5% or 10% of a composition of the invention for 24 hr at which time cell lysates were prepared for measurements of SOD activity using a commercially available kit (SOD activity, cat# 900-157) according to manufacturer's (Assay Designs, Ann Arbor, M1) directions. Cell culture medium was assayed for SOD activity in parallel. Limited trials with smaller concentrations of a composition of the invention < 1 % and murine epidermal cells were also attempted.

Results of First-Attempt Methods to Determine SOD activity for high serum a composition of the invention concentration: Diluted lysates showed a marginal increase in enzymatic activity associated with treatment with a composition of the invention. Changes in enzymatic activity were marginal in the initial range of 5-10% a composition of the invention (final concentration, v/v). The data represent the first attempt to measure SOD activity using primary HMVEC-L cells treated with a composition of the invention. It is feasible that the lack of SOD activity associated with 5-10% a composition of the invention might be related to non-specific inhibition at high dose. The primary concern is that we have little understanding of the primary human HMVEC-L cell model and cannot determine whether these cells are optimal for investigating antioxidant defense regulation induced by a composition of the invention. For example, ascorbic acid, known to break down certain redox signaling complexes in A a composition of the invention, is supplemented into the medium and it is feasible that some modification of the medium formula (such as omission of ascorbic acid for short periods of time defined empirically) could produce more optimal conditions for detecting antioxidant defense regulated by a composition of the invention. Initial efforts to serum-starve these cells, as one approach to increase sensitivity and optimize the model, were unsuccessful and resulted in extensive cell death over 24 hours, indicating that the cells are dependent on the growth factors supplemented in the cell culture medium to maintain cell viability. If we interpret the initial a composition of the invention concentrations (5-10%) to be high (based on inhibition of medium enzymatic activity and cell proliferation), then it is possible that the marginal increase in enzymatic activity associated with cell lysates observed here may not accurately reflect antioxidant defense regulation possibly occurring at lower concentrations. The use of an *in vitro* model system with a well defined and robust NRF2-regulated antioxidant defense response would help address some of these uncertainties. In retrospect, we have observed that a lower concentration of a composition of the invention (1%) induces the nuclear translocation of the NRF2 transcription factor. In addition, the 24 hr time point was chosen for the initial screen as a general time point for *in vitro* investigations that would capture transcriptional regulation; however, this time point was not optimal.

Results of Further Investigations into SOD enzymatic activity at low composition of the invention concentrations (< 1%): It was found in another investigation that NRF2 nuclear translocation (data and results are in the following sections), took place at low doses of a composition of the invention (less than 1 %) and elicited peak SOD antioxidant activity at about 30 to 120 minutes after exposure. Thus when SOD antioxidant activity was measured due to low-concentration composition of the invention exposure at 30 to 120 minute time points, results similar to the GPx enzymatic activity were seen both with murine epidermal (JB6) cells and serum-starved HMVEC-L cells at a time point 90 to 120 minutes. A 500% increase in peak SOD enzymatic activity was estimated over a short 120 minute term, with 95% confidence.

Experimental Methods Used to Determine Nuclear Translocation of NRF2 in HMVEC-L Cells and Western Blot Verification: HMVEC-L cells were again thawed and maintained according to manufacturer's directions. The culture medium contained epidermal growth factor, hydrocortisone, GA-1000, fetal bovine serum, vasoactive endothelial growth factor, basic fibroblast growth factor, insulin growth factor-1 and ascorbic acid in randomly cycling cultures. Ascorbic acid was withheld from serum-starved cultures.

HMVEC-L Cell cultures in both normal random cell cycles and in serum starvation were exposed to high-concentration (5-20%) and low-concentration (1%) ASEA in the serum medium and analyzed in conjunction with cultures exposed only to phosphate buffered saline solution (PBS), as a negative control. At time points of 30, 60, 90, and 120 minutes, aliquots of cells from each of the cultures were placed under a fluorescent microscope, stained by a fluorescent dye designed to tag the NRF2 transcription factor along with the DAPI fluorescent nuclear stain that aids the computer software to find the nuclei. Computer automated imaging techniques were used to determine the relative degree of nuclear accumulation of NRF2 via fluorescent analysis over several cells. NRF2 regulates the transcription of a number of phase II antioxidant defense enzymes and raises the possibility that additional antioxidant defense enzymes, such as glutathione transferase, may be expressed through exposure to ASEA. Thus the accumulation of NRF2 into the nucleus, as seen visually in the microscope images, is an indicator of increased antioxidant expression in the cells.

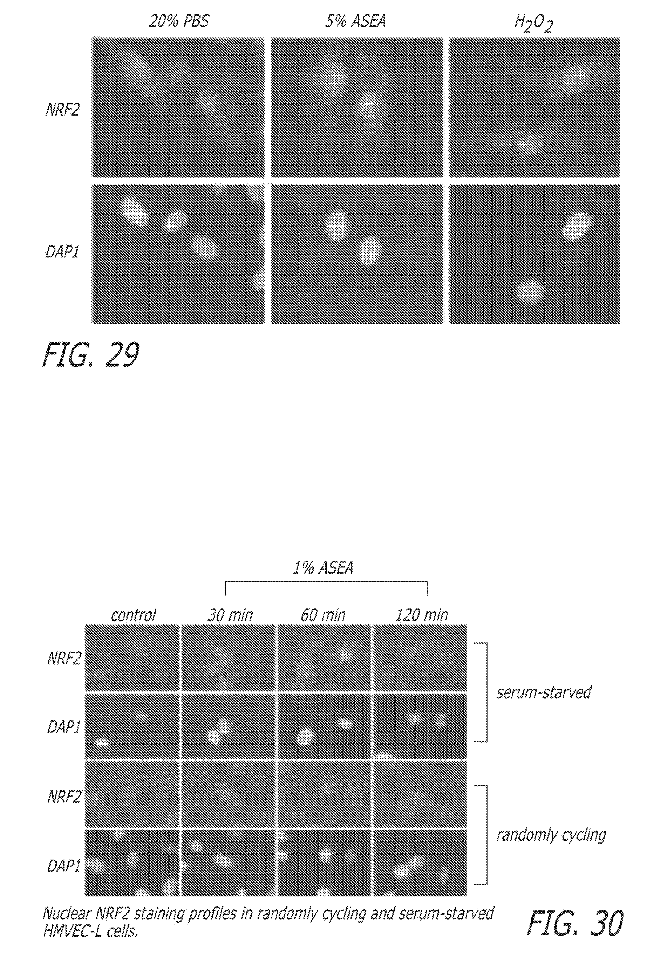

Results of HMVEC-L Nuclear Accumulation of NRF2: Initial screen of human endothelial cells suggests a subpopulation of cells showed increased nuclear staining pattern (focal) following treatment with high-concentration of a composition of the invention. The positions of nuclei are indicated by DAPI stain in lower panel. Foci appear brighter in a composition of the invention stimulated cells which indicates higher level of NRF2 transcription factor in the nucleus. H₂O₂ was used as positive control. This effect was difficult to quantify based on nuclear staining pattern. (Figure 29).

Typical cell images are shown below for indicated cell cultures exposed to low concentrations of a composition of the invention. Accumulation of NRF2 into the nucleus was clearly seen in serum-starved cell cultures exposed to low-concentrations of a composition of the invention. Automated analysis revealed strong time- dependent nuclear accumulation of NRF2 in serum-starved cells, relative to the negative control, at the 30 and 60 minute time points (Figure 30).

The nuclear staining profile was qualitatively different from the cells maintained in optimal growth medium (randomly cycling group). There was weak qualitative nuclear accumulation of NRF2 induced by exposure to a composition of the invention in these cells at 30, 60 and 120 minute time points, and yet the effect was not nearly as pronounced as in the serum-starved cultures. However, serum-starvation induced significant cell death complicating interpretation of the data. The trends appeared weak and require validation by Western Blot.

Experimental Methods for Western Blot Validation of NRF2 Nuclear Accumulation: HMVEC-L were treated with 1% of a composition of the invention, nuclear extracts were separated through centrifugal differentiation from the extra-nuclear cytosol at 30, 60 and 120 min and subjected to Western Blot analysis for NRF2. In the Western blot experiment the extra-nuclear fraction was probed for phosphorylated proteins using a combination of anti-phospho serine, threonine and tyrosine antibodies. Virtually all cellular processes are regulated by posttranslational modifications and protein phosphorylation is a prevalent mechanism. Observable changes in protein phosphorylation can lead to a mechanistic understanding of the cellular processes perturbed by compositions of the invention and provide a defined endpoint to better define dose-dependent regulation of cell function by compositions of the invention *in vitro,* as well as provide a potential candidate molecular marker that may be used to provide *in vitro-in vivo* correlates. Hydrogen peroxide (H₂O₂) was included as a positive control for oxidant damage.

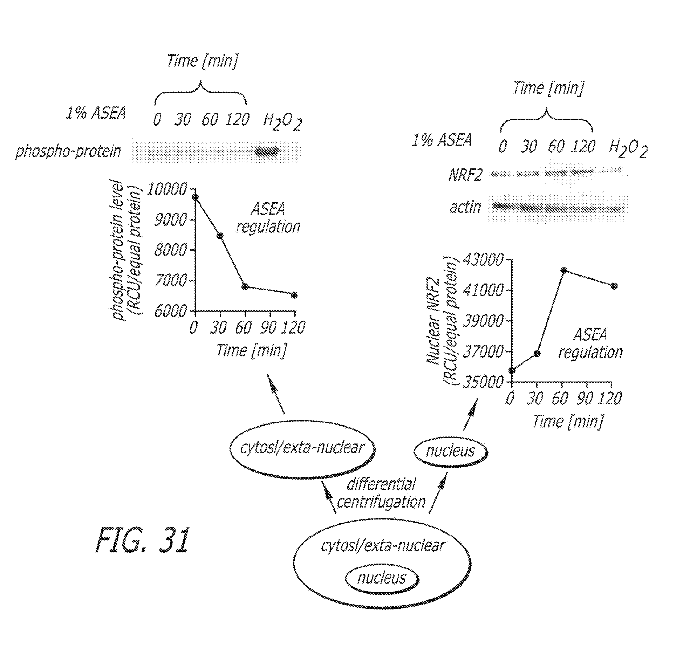

Results for Western Blot Validation of NRF2 Nuclear Accumulation: NRF2 levels were increased in a time-dependent fashion in nuclear extracts prepared from HMVEC-L cells treated with 1 % ASEA. H₂O₂ (30 min) did not increase nuclear NRF2 levels. In contrast, when protein phosphorylation was examined in the extra-nuclear fraction (separated from nuclei by differential centrifugation) we observed a single band by Western blot analysis and this is likely due to the dilution of the extra-nuclear fraction during the cell fractionation process (other phosphorylated proteins are obviously present but are below detection limits under these conditions) or specificity of the anti-phospho-antibodies used was insufficient to detect a broad range of phosphorylated proteins. However, we did observe a marked increase in the phosphorylation of the protein detected following H₂O₂ treatment, indicating that this phosphorylation event is highly sensitive to redox regulation or activation of protein kinase/deactivation of protein phosphatase activities subsequent to oxidative damage. Treatment of cells with 1 % of a composition of the invention decreased phosphorylation levels associated with this protein in a time-dependent fashion (Figure 31).

Reductions in phosopho-protein regulation in extra-nuclear fractions were seen along with strong time-dependent NRF2 accumulations in the nuclear fractions, indicating clear time-dependent up-regulation of antioxidant expression.

At this point it is worth mentioning that NRF2 activity has been clearly detected in conjunction with exposure to a low-concentration of a composition of the invention without the normal prior NF-kB activity. This suggests that phase II antioxidant defense mechanisms have been stimulated without the normal prior phase I toxic response. This behavior has no precedent or is extremely rare. It appears from the data that compositions of the invention are able to stimulate antioxidant expression without ever eliciting a prior low-level phase I toxic response.

Experimental Methods to Determine Proliferation of Murine (JB6) Cells and HMVEC-L Cells and LDH Activity with Exposure to ASEA: HMVEC-L cells were treated with 5-20% ASEA for 72 hr and cell number was determined using a Coulter Counter. Control (0 concentration group) was treated with 20% PBS. Serum LDH levels were also measured as an indicator of cell culture viability at 0 to 20% concentration of the compositions of the invention I serum concentrations. Recall that lower serum LDH concentrations indicate less cell membrane failure. Similar experiments were performed for murine (JB6) epidermal cells.

Results for Proliferation of Murine and HMVEC-L cells and LDH activity (Figure 41, 42): The initial *in vitro* screen indicates that high-concentrations of compositions of the invention in serum may inhibit cell proliferation (for both murine epidermal cells [JB6] and primary human lung microvascular endothelial cells [HMVEC-L]) in the concentration range of 5-20%. In this concentration range we also observed direct inhibition of LDH enzymatic activity. The data are somewhat contradictory as the decreasing cell counts indicate cell death, yet lower serum LDH levels indicate higher cellular membrane integrity. At the highest concentration tested (20% v/v), cell proliferation was inhibited by approximately 20%.

The mechanism behind reduced proliferation cannot be deduced and could be related to interference with growth factor responsiveness or other possible interpretations such as enhanced programmed death (apoptotic response) for damaged cells. It is noteworthy that high serum concentrations of composition of the invention for *in vitro* enzymatic enhancement studies is not optimal, it is possible that the initial screens underestimated or even missed antioxidant defense (SOD) regulation by a composition of the invention and thus indicate that low-concentration (<1 %) compositions of the invention and/or short exposure times should be employed for such purpose.

Further studies were done that investigated the action of stressed cells upon exposure to compositions of the invention; the source of stress resulting from a variety of chemical and environmental stressors. These investigations offer clues for the possible mechanisms.

Experimental Methods to Determine cell viability of HMVEC-L exposed to various mixtures of Cachexin stressor and high-concentration compositions of the invention: HMVEC-L cultures with normal random cell cycles (pS) and cultures approaching confluence (A2), which are generally less sensitive to Cachexin, were infused with escalating concentrations of Cachexin stressor (0-5 ng/mL). These cultures had been pretreated with either a 10% PBS control or 5-10% concentration of a composition of the invention for 24 hours. Two indicators for cell viability were employed. Serum LDH levels were obtained as an indication of membrane integrity and Neutral Red dye was used as an indication of lysosomal integrity. Recall that as cell membranes fail, LDH is released into the serum medium. Lower quantities of LDH indicate higher cell viability. The integrity of lysosomes, necessary for viable cell function, are measured by absorption of Neutral Red dye stain. Higher quantities of Neutral Red absorbance indicate higher cell viability.

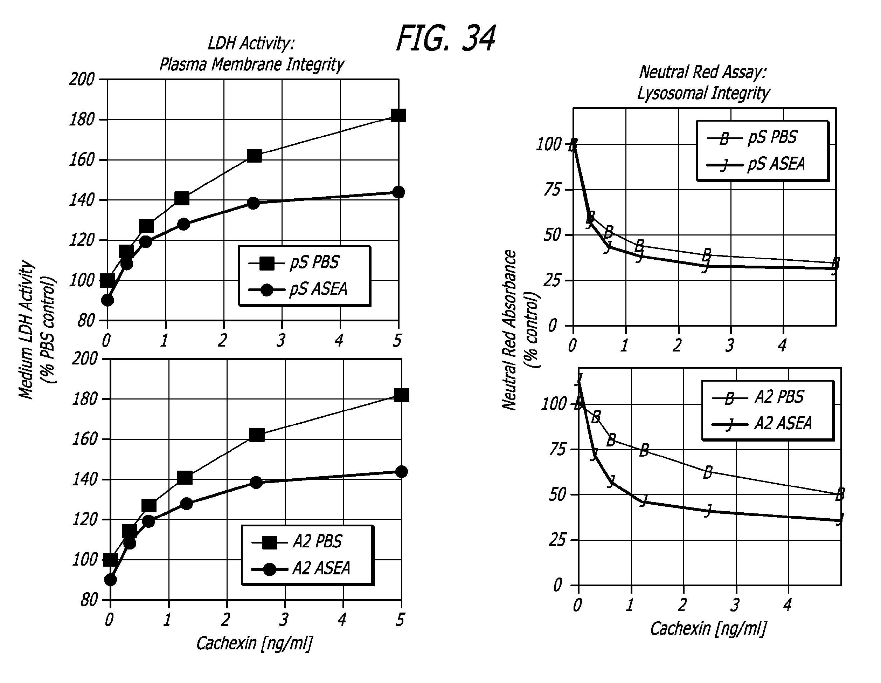

Results of HMVEC-L viability exposed high-concentration composition of the invention and to escalating amounts of Cachexin stressor (Figure 34): Both confluent (A2) and normal (pS) HMVEC-L cultures exhibited up to 30% improvement (relative to PBS controls) in LDH levels related to exposure to compositions of the invention after acute (up to 5 nm/mL) Cachexin insult. The LDH data suggest that HMVEC-L cells stressed by Cachexin are less likely to die due to cell membrane failure after being exposed to compositions of the invention.

Behavior of lysosomal integrity in HMVEC-L cells as measured by Neutral Red absorption exhibited behavior dependent on cell culture phase. As expected, the confluent (A2) cells in the PBS control were much less sensitive to Cachexin insult than cells in the PBS control normal random phase (pS) culture; this is evidenced in the 5 ng/mL Cachexin data: Lysosomal levels in A2 cells dropped only 50% compared to 70% in the pS culture. Exposure of the normal (pS) cultures to compositions of the invention made little difference in lysosomal integrity under similar Cachexin insult, yet exposure of confluent (A2) cell cultures to ASEA made them much more sensitive to Cachexin insult, regressing to behavior similar to that exhibited by the normal more sensitive (pS) cells.

This is the first evidence presented that suggests that exposure of abnormal (Cachexin-insensitive) HMVEC-L cells to compositions of the invention can make them more sensitive. The data suggest that confluent (A2) cells stressed by Cachexin are more likely to die when exposed to compositions of the invention, these abnormal cells when exposed to ASEA exhibit closer to normal behavior in the presence of Cachexin. This behavior was initially unexpected as the hypothesis of the experiment was that compositions of the invention would help cells protect themselves against toxic insult. As it turns out, it appears that compositions of the invention exposure only helps normal healthy cells to protect themselves against oxidative insult and yet seems not to help cells protect themselves against Cachexin. Exposure to compositions of the invention may even help facilitate the death of the stressed cells that are close to the end of their normal life cycle. Incidentally, the normal role of Cachexin in the tissues is to facilitate the death and replacement of damaged cells.

Experimental methods to determine the compositions of the invention concentration-dependent response of A2 and pS phase HMVEC-L cells to Cachexin insult: HMVEC-L cell cultures, prepared in two phases, in the confluent end-of-life-cycle A2 phase (a phase typically insensitive to Cachexin insult) and in the normal random cycle pS phase were exposed for 24 hours to serum concentrations (v/v of 2.5%, 5%, 10%, 15% and 20%) of either the PBS control or a composition of the invention. Cachexin responsiveness was then determined by monitoring LDH activity in both the intracellular cytosol and in the surrounding growth media. Recall that increased LDH activity in the growth media indicates cell membrane rupture and death (LDH release) and the decrease of intracellular LDH activity indicates loss of cellular integrity. Thus the cell cultures that are responsive to Cachexin insult would experience an increase in medium LDH activity and a decrease in intracellular LDH activity.

LDH activity in untouched cell culture controls were compared to that of cell cultures insulted with 5 ng/mL Cachexin for each composition of the invention concentration considered. The concentration dependence of compositions of the invention was then graphed against LDH activity for each insulted culture and control.

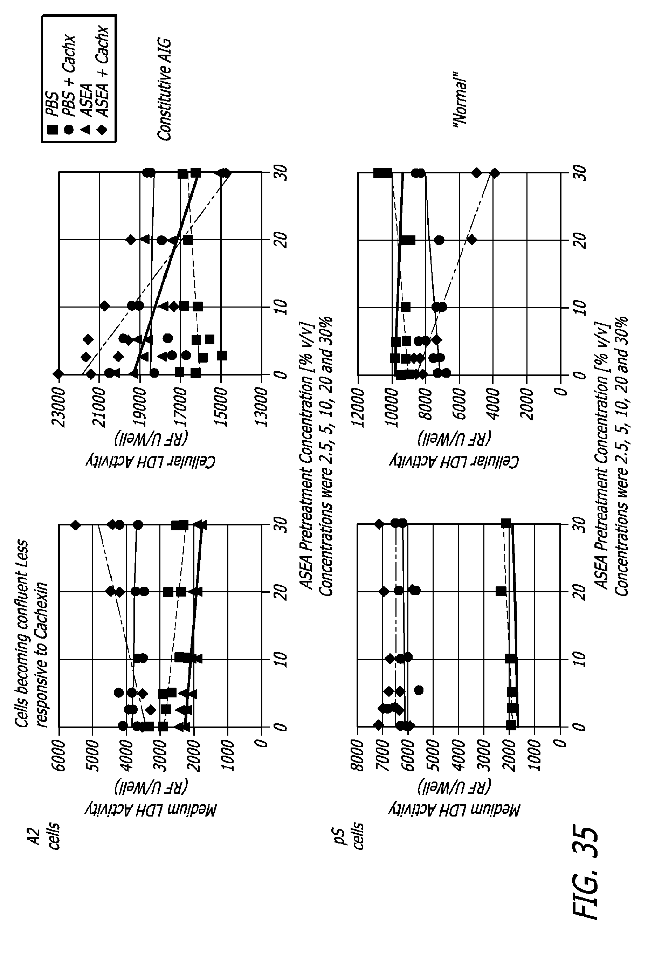

Results of concentration-dependent response of HMVEC-L cells to Cachexin insult (Figure 35): Relative to the PBS control, the Cachexin response for the normal pS cells was much smaller than expected. Only slight decreases in cell membrane integrity were seen in the PBS control cultures and the intracellular LDH activity remained the same. With exposure to compositions of the invention, by itself, the normal pS cell cultures suffered a slight decrease in overall cellular integrity and increase in cell death. It should be noted that since the large expected response of the control pS cells to Cachexin was not manifest, it is probable that the pS cell cultures used in this investigation were nearing a confluent or nonresponsive state.

There was, however, a clear response when Cachexin insult was added to the pS cell cultures exposed to various composition of the invention concentrations, cultures demonstrated a clear loss of intracellular LDH function and integrity. However, the accompanying indication of cell death was not seen. This seems to indicate that the "normal pS" cells were made more sensitive to Cachexin reception by composition of the invention exposure, yet not brought completely to the point of cell death.

The A2 cell culture response was very clear. Composition of the invention exposure, even without Cachexin, seemed to cause loss of intracellular LDH integrity, though it did not affect cell death. However, when Cachexin insult was applied to such A2 cultures, composition of the invention exposure clearly amplified the Cachexin reception rapidly decreasing cellular function and there were also clear indications of concentration dependent cell death. There is strong evidence that exposure to compositions of the invention increases Cachexin responsiveness in the A2 cell cultures.

The results imply that exposure to compositions of the invention significantly increases Cachexin responsiveness in A2 and borderline pS HMVEC-L cell cultures. Of possible interest, exposure to compositions of the invention alone might decrease integrity of cellular LDH activity in A2 type cells; recall that zero toxic response was detected in randomly cycling cells even under large concentrations, so effects due to toxicity are not expected in normal cells. It appears that exposure to compositiosn of the invention may tend to accelerate the removal of non-responsive confluent cells. This is evidently true when Cachexin is present. These results might also bear on the observations that exposure to compositions of the invention seemed to diminish cell proliferation in high concentrations. No such trend was tried for low-concentration exposure. Note that it is difficult to discount the possibility that high-concentration effects might simply be artifacts due to the interference of compositions of the invention with the growth medium.

Experimental methods to determine effects of 5-10% composition of the invention exposure to cells stressed by radiation and serum starvation: Murine (JB6) cell cultures were subjected to high-level radiation exposure (X-rays) and, in a separate investigation, cultures were subject to serum starvation of growth factors for 24 hours. The cells were then exposed to 5-10% ASEA exposure as means to determine the effect of composition of the invention exposure on such stressed cells. Cell counts were taken before and after composition of the invention exposure.

Results of effects of 5-10% composition of the invention exposure on radiation and serum-starved murine cells: Quantitative analysis was not compiled for these experiments. Qualitative analysis, however, reveals results that might be of some interest. For the radiation-damaged culture, immediate cell death was observed for more than half of the culture upon exposure to composition of the invention. No further cell-death was seen thereafter. Upon inspection under a microscope, the remaining living cells appeared normal and healthy. It appears that exposure to a composition of the invention may have helped accelerate cell death among the more seriously damaged cells and allowed for the survival of healthy or repairable cells.

For serum-starved cell cultures similar observations were made, except the cell death was not nearly as severe, amounting to less than roughly a 20% loss. Surviving cells appeared to be very robust and viable. Similar losses, however, were also seen in serum starved cultures that were not exposed to compositions of the invention in later experiments.

A better understanding of the bioactivity of a certain mixture of redox signaling molecules has been determined from *in vitro* studies involving direct contact of compositions of the invention with viable living HMVEC-L human cells and murine epidermal JB6 cells. Five specific objectives were pursued to determine:
1) *In vitro* toxicity (based on NF-kB, P-Jun translocation)
2) Effects on antioxidant efficacy (for GPx and SOD)
3) Effects on antioxidant transcriptional activity (NRF2)
4) Effects on cell proliferation and viability (cell counts)
5) Effects on stressed cells (Cachexin, radiation, starvation)

No toxic response was observed for any healthy cell culture in normal random phases (HMVEC-L or JB6) upon exposure to high concentration compositions of the invention (up to 20%) of serum. Two methods were used to determine toxic response, the translocation and accumulation of NF-kB and P-Jun in the nuclei. Both of these methods are known to be sensitive to low-levels of toxicity, as verified by the positive control. A complete lack of toxic indication and/or inflammatory cytokines was observed.

An 800% increase in GPx antioxidant efficacy in HMVEC-L cells was seen after 24 hours exposure from low-concentration composition of the invention (no concentration dependence seen). A transitory increase of up to 500% was seen in SOD antioxidant efficacy between 30 to 90 min. again after exposure to a low-concentration of a composition of the invention (<1%). In both cases, the low concentrations of compositions of the invention were comparable to blood concentrations possible from oral dosing, though data is not available to confirm this. Concentration dependence at very low concentrations might be seen if such was carefully investigated.

Exposure to high-concentration compositions of the invention, in comparison, elicited only a small relative increase in GPx antioxidant efficacy that was not concentration dependent. An increase in SOD efficacy was not seen for either high-concentration compositions of the invention or after long (24 hr) exposures. In subsequent investigations, this information will be used to determine optimal concentrations and time points to study concentration dependence (<0.1 % and 0-120 minutes).

Studies examining the nuclear translocation of redox responsive transcription factors suggest that compositions of the invention at a lower concentration (less than 1%) induces a 20-30% increase in the nuclear translocation of the NRF2 transcription factor in HMVEC-L cells that appears to be transient (30-60 min). We also observed that a composition of the invention induced a parallel decrease in the phosphorylation of an extra-nuclear protein whose phosphorylation status is clearly increased in response to hydrogen peroxide treatment, consistent with an antioxidant mode of action.

Serum-starving HMVEC-L cells, as an approach to increase sensitivity, significantly increased the nuclear NRF2 signal induced by composition of the invention (1%). However, serum-starvation induced significant cell death complicating interpretation of the data.

Cellular proliferation for both HMVEC-L and JB6 cell types (determined from cell counts) was inhibited by high concentrations (5-20% v/v) of exposure to compositions of the invention. The HMVEC-L inhibition was clearly concentration dependent, with a 20% loss of cell count at 20% ASEA concentration. In contrast to decreased proliferation, serum LDH levels significantly decreased with compositions of the invention concentration between 5-20%, indicating increased cell membrane integrity. The results seem to indicate that cellular proliferation is decreased while cell membrane viability is increased at high concentrations. The mechanism behind such behavior cannot be deduced from the data, yet further evidence will be seen in the next section.

The response of HMVEC-L cells when stressed with Cachexin depends upon cell phase. Normal randomly cycling HMVEC-L cells (pS) exhibited typical behavior when stressed with Cachexin: exhibiting decrease in cell viability accompanied by cell death. Confluent end-of-life-cycle (A2) and borderline HMVEC-L cells, as expected, were less sensitive to Cachexin insult, exhibiting less pronounced decreases in cell viability and less cell death.

Exposure to compositions of the invention caused no significant change in the response of the normal random cycling pS cells to Cachexin (showing similar loss of cell viability and cell-death). However, A2 cell cultures exposed to a composition of the invention exhibited increased sensitivity to Cachexin, restoring behavior similar to that of normal cells. This behavior was reinforced as concentration dependence was examined. Borderline A2 cells, exhibiting a relatively small Cachexin response, and A2 cells that are normally insensitive to Cachexin insult, exhibited a much stronger response to Cachexin when exposed to compositions of the invention, both in decrease in viability and increased cell death.

It appears that exposure to compositions of the invention causes increased rates of A2 cell death, enhancing the natural reception of Cachexin in such end-of-life-cycle cells. Yet exposure to composition of the invention is not expected to cause any change in normal cell viability.

Cachexin is normally secreted to instigate cell death in damaged or dysfunctional tissues, allowing surrounding healthy cells to divide and fill in voids. Thus, increasing the sensitivity to Cachexin in dysfunctional cells may help accelerate such a process and is not always deleterious.

Acceleration of cell death was also seen in tissues that were stressed with radiation and serum-starvation associated with exposure to compositions of the invention.

The infusion of a certain balanced mixture of redox signaling molecules using compositions of the invention into viable HMVEC-L and JB6 cell cultures has been seen to elicit distinct bioactivity. No indications of toxicity or the expression of inflammatory cytokines were observed and yet there was increased antioxidant and protective enzyme expression (as evidenced by increased nuclear NRF2) and greatly increased efficacy for the two master antioxidants, GPx and SOD. This behavior suggests that infusion with compositions of the invention might tend to induce and enhance oxidative defense mechanisms without inducing toxic or inflammatory responses in such cells. Such action is unprecedented or extremely rare. Normally, low-level toxicity induces slight oxidative stress and inflammatory response which in turn induces oxidative defense and cell repair mechanisms. It would be of interest to determine concentration dependency of this effect with ultra-low-concentration infusions of compositions of the invention.

The induction of cell death in cultures of dysfunctional, stressed or damaged cells by infusion of compositions of the invention should also be explored. Natural healing processes involve a repair or replace mechanism by which marginally damaged cells are repaired, when possible, or undergo apoptosis, programmed death, if they cannot be repaired and then are replaced through mitosis of healthy neighboring cells. It is fairly evident that infusion of composition of the invention, of itself, is not causing direct stress to exposed cells, however, it might tend to increase the efficiency of certain cytokine "death domain" messengers (Cachexin) that are designed to induce cell death in dysfunctional or damaged cells. The nuclear translocation of NRF2 can be considered part of the phase II oxidative defense response which includes expression of antioxidants, DNA repair molecules and other known repair mechanisms.

Apoptosis is part of the replace mechanism when cells have undergone unrepairable damage and must be removed and replaced. Both antioxidant defense and apoptotic mechanisms are central to normal tissue repair and regeneration. Redox signaling is involved in several of the pathways, such as p53 gene expression, that can determine whether a cell undergoes apoptosis or not. Chronic oxidative stress tends to favor cell death. Certainly the presence of Cachexin and other death domain messengers favor cell death. The observation that infusion with compositions of the invention enhances Cachexin reception might indicate that infusion with compositions of the invention also might serve to enhance reception of messengers in the signaling process that determines whether defense, repair or replace mechanisms are activated.

### Example 9

### Treatment of Type 2 Diabetes

A 42 year old man diagnosed with type 2 diabetes is treated by intravenously injecting a combination product comprising a PPAR inhibitor (an eicosanoid) and a redox signaling agent (RXN) composition of the invention. After about 1 week, the patient maintains a more regular glucose profile with minimal glucose excursions as compared to prior to treatment.

### Example 10

### Treatment of Type 1 Diabetes

A 22 year old woman diagnosed with type 1 diabetes is treated by intravenously injecting a combination product comprising a PPAR inhibitor (a thazolidinedione) and a redox signaling agent (RXN) composition of the invention. After about 1 week, the patient maintains a more regular glucose profile with minimal glucose excursions as compared to prior to treatment.

### Example 11

### Treatment of Cancer

A 31 year old woman diagnosed with cancer is treated by intravenously injecting a combination product comprising a PPAR inhibitor (a fibrate) and a redox signaling agent (RXN) composition of the invention. After about 1 month, the patient shows a regression in her symptoms.

### Example 12

### Treatment of Type 2 Diabetes

A 72 year old man diagnosed with type 2 diabetes is treated by orally administering a combination product comprising a PPAR inhibitor (an eicosanoid) and a redox signaling agent (RXN) composition of the invention. After about 1 week, the patient maintains a more regular glucose profile with minimal glucose excursions as compared to prior to treatment.

### Example 13

### Treatment of Type 1 Diabetes

A 61 year old woman diagnosed with type 1 diabetes is treated by orally administering a combination therapy comprising a PPAR inhibitor (a thazolidinedione) and a redox signaling agent (RXN) composition of the invention. After about 1 week, the patient maintains a more regular glucose profile with minimal glucose excursions as compared to prior to treatment.

### Example 14

### Treatment of Cancer

A 31 year old woman diagnosed with oral cancer is treated by orally administering a combination product comprising a PPAR inhibitor (a fibrate) and a redox signaling agent (RXN) composition of the invention. After about 1 month, the patient shows a regression in her symptoms.

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain embodiments of this invention are described herein, including the best mode known to the inventors for carrying out the invention. Of course, variations on these described embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

In closing, it is to be understood that the embodiments of the invention disclosed herein are illustrative of the principles of the present invention. Other modifications that may be employed are within the scope of the invention. Thus, by way of example, but not of limitation, alternative configurations of the present invention may be utilized in accordance with the teachings herein. Accordingly, the present invention is not limited to that precisely as shown and described.

The following clauses specify aspects of the invention:
1. A composition comprising at least one redox signaling agent (RXN) and a PPAR agonist.
2. The composition of clause 1, wherein the PPAR agonist comprises a Free Fatty Acid (FFA).
3. The composition of clause 1, wherein the PPAR agonist comprises an eicosanoid.
4. The composition of clause 1, wherein the PPAR agonist comprises a thazolidinedione.
5. The composition of clause 1, wherein the PPAR agonist comprises a fibrate.
6. The composition of clause 1, wherein the PPAR agonist comprises a dual agonist.
7. The composition of clause 6, wherein the dual agonist comprises aleglitazar.
8. The composition of clause 6, wherein the dual agonist comprises muraglitazar.
9. The composition of clause 6, wherein the dual agonist comprises tesaglitazar.
10. The use of a composition comprising RXN and a PPAR agonist in the treatment of a PPAR-mediated disease.
11. The use of clause 10, wherein the PPAR-mediated disease is diabetes mellitus.
12. The use of clause 11, wherein the diabetes mellitus is type 1 diabetes.
13. The use of clause 11, wherein the diabetes mellitus is type 2 diabetes.
14. The use of clause 10, wherein the PPAR-mediated disease is obesity.
15. The use of clause 10, wherein the PPAR-mediated disease is cancer.
16. The use of a composition comprising at least one RXN in the treatment of cancer, comprising a first therapy which activates a PPAR pathway and at least one other agent wherein the at least one other agent does not activate a PPAR pathway.
17. The use of clause 16, wherein the at least one other agent comprises radiation.
18. The use of clause 16, wherein the at least one other agent comprises a chemotherapeutic agent.
19. The use of a composition comprising at least one RXN in the treatment of cancer, comprising a first therapy which mobilizes Free Fatty Acids (FFAS) and at least one other agent wherein the at least one other agent does not mobilize FFAs.
20. The use of clause 19, wherein the at least one other agent comprises radiation.
21. A method of treating an oxidative stress related disorder comprising:
   administering a composition including at least one species selected from O₂, **H**₂, Cl₂, OCr, HOC1, NaOCl, HClO₂, ClO₂, HClO₃, HClO₄, H₂O₂, Na⁺, Cl⁻, **H**⁺, **H**⁻, OH⁻, O₃, O₄*⁻, ¹O, OH*⁻, HOCl-O₂*⁻, HOCl-O₃, O₂*⁻, HO₂*, NaCl, HCl, NaOH, water clusters, or a combination thereof to a patient experiencing oxidative stress; and
   treating the oxidative stress related disorder.
22. A method of treating a reduced mitochondrial DNA disorder comprising:
   administering a composition including at least one species selected from O₂, **H₂,** Cl₂, OCr, HOC1, NaOCl, HClO₂, ClO₂, HClO₃, HClO₄, H₂O₂, Na⁺, Cl⁻, **H**⁺, **H**⁻, OH⁻, O₃, O₄*⁻, ¹0, OH*⁻, HOCl-O₂*⁻, HOCl-O₃, O₂*⁻, HO₂*, NaCl, HCl, NaOH, water clusters, or a combination thereof to a patient experiencing the reduced mitochondrial DNA disorder;
   increasing mitochondrial DNA density; and
   treating the reduced mitochondrial DNA disorder.
23. The method of clause 22, wherein the reduced mitochondrial DNA disorder is sacropenia, diabetes, Alzheimer's disease, Parkinson's disease, neurological disease, muscle loss due to aging, obesity, or cardiovascular disorders.

## Claims

1. A method for producing a stable, electrolyzed saline solution, said method comprising:
preparing a saline solution having a ratio of about 10.75g of sodium chloride per 1 gallon (3.78541 litres) of water;
inserting within the saline solution an inert anode and a spaced apart corresponding inert cathode associated with a power source;
controlling the temperature of the saline solution to regulate relative concentrations of resulting species during electrolysis;
circulating the saline solution to maintain a flow of the saline solution between the anode and cathode;
applying a voltage potential selected from within a voltage range of 0 volts to approximately 12 volts between the cathode and the anode sufficient to produce a mixture of chemically reduced and oxidized molecules, the mixture including hypochlorous acid, hypochlorites, hydrogen gases, superoxides, ozone, chloride ions, hydroxides, and other forms of reactive oxygen species; and
mirroring molecular compositions of native salt water compounds found in and around human cells, said molecular compositions containing stable reactive oxygen species, said mirroring comprising one or more of the following:
adjusting a temperature of the saline solution;
adjusting anode and cathode spacing;
adjusting a saline solution circulation rate; and
optimization of the voltage.

2. The method of claim 1, wherein the step of regulating the temperature of the saline solution further comprises a step for maintaining a desired temperature of the saline solution at 4.8°C.

3. The method of claim 1, wherein the voltage potential is approximately 9 volts.

4. The method of claim 1, wherein the inert cathode is positioned coaxially in relation to a position of the inert anode.

5. The method of claim 1, wherein the inert cathode comprises a base metal selected from the group consisting of copper, aluminum, titanium, rhodium, platinum, silver, gold, iron, a combination thereof, and an alloy such as steel or brass.

6. The method of claim 1, wherein the inert anode and the spaced apart corresponding inert cathode are separated by a distance of are spaced a surface of the inert anode, wherein the spacing is from approximately 2.5 cm to not more than 50 cm.

7. The method of claim 1, further comprising a step for maintaining a pH of the saline solution between approximately 6.8 and approximately 8.2.
